# EUROPEAN PATENT APPLICATION

(11) **EP 4 020 490 A1**
(43) Date of publication of application: **29.06.2022**
(21) Application number: 20854468.4
(22) Date of filing: 20.08.2020
(51) Int. Cl.: G16H 20/00, G06Q 50/22

(54) **HEALTHCARE DEVICE, SYSTEM, AND METHOD**

(30) Priority: 22.08.2019 SG 10201907782V
(71) Applicant: Shining Inc., Otaru-shi, Hokkaido, 047-0156 (JP)
(72) Inventor: OSANAI, Kaori, Otaru-shi Hokkaido 047-0156 (JP)
(74) Representative: Ipey
(86) International application number: PCT/JP2020/031508
(87) International publication number: WO 2021/033755

(57) **Abstract**

[Problem] To provide a healthcare device, healthcare system, and healthcare method. [Solution] A healthcare device 2 is configured comprising: a reception unit 21 that receives first data related to at least one condition associated with a first user; a control unit 22 that determines second data on the basis of at least the first data and healthcare data; and an output module 23 that outputs the second data. The second data comprises first information and second information, said first information comprising one or more health conditions of the first user, and said second information comprising a degree of urgency with respect to each of said one or more health conditions.

## Description

### TECHNICAL FIELD

The present disclosure relates to a healthcare device, system, and method, more particularly, to a triage device that selects a necessary measure on the basis of a health condition of a user, system, and method.

### BACKGROUND

An object of the following discussion about the background of the disclosure is to facilitate understanding the present disclosure. The discussion shall be construed as not admitting that any of the mentioned documents have been published and that a part of general knowledge of a person skilled in the art has been announced as a preferential date in a known or any jurisdiction.

A healthcare system (i.e. a triage system) that determines a degree of urgency by a call between a user(patient) and an operator is proposed (Patent Literature 1). In this healthcare system, the operator can decide a degree of urgency related to a measure to the user(patient) on the basis of information that is provided from the user(patient).

### PRIOR ART DOCUMENTS

### PATENT LITERATURE

Patent Literature 1: Japanese Unexamined Patent Application Publication No. 2013-41479

### SUMMARY

### THE PROBLEM TO BE SOLVED BY THE INVENTION

The art of Patent Literature 1 has the following problems.

Firstly, an operator inputs provided information that is provided from a user(patient). However, for example, when the content of the provided information is vague, in order to make the content of the provided information easy to understand, the operator may add subjective evaluation of the operator on the provided information, which the operator inputs in the healthcare system.

Secondly, when the user(patient) himself/herself doesn't remember his/her personal history related to his/her own anamnesis, and the user(patient) provides information to the operator, at least a part of the personal history related to the anamnesis of the provided information that the user(patient) provides may be missing.

As the art of Patent Literature 1 has these two problems, at least one of diseases that the system presents as a possible disease may be either inaccurate or insufficient, or may be both. The aforementioned two problems need improving, in order to enable the system to decide quickly and accurately a possible disease and a degree of urgency necessary for treating the possible disease to provide an appropriate healthcare measure to a person concerned(such as a doctor, a nurse, an operator, a patient, etc.).

The present invention has been made in view of such circumstances, and an object of the present invention is to provide a healthcare device, a system, and a method: enabling the healthcare device to receive information that excludes subjectivity of an operator as much as possible; by adding such information and information covering more surely anamnesis from which the patient has suffered, grasping a health condition and a degree of urgency of the patient exemplified by a predicted disease, and a medical examination department of a specialized category related to a selected medical examination or the like thereby; and capable of selecting more correctly a necessary measure exemplified by a decision or the like of an inspection necessary for the patient, a medical treatment policy, a degree of urgency of a consultation, an order of a medical treatment for the patient, an order of an emergency transport, and a designated facility, on the basis of the health condition and the degree of urgency of the patient that are grasped.

### MEANS FOR SOLVING THE PROBLEMS

As a result of diligent study of the aforementioned problems, the inventors have found that it is possible to achieve the aforementioned objective by receiving healthcare data associated with a user to decide a health condition of the user and a degree of urgency thereof, on the basis of the healthcare data or the like that is received. Specifically, the present invention provides the following.

Throughout this document, unless the context otherwise requires, the terms such as "comprising," "consisting of," "having," or the like shall be construed as non-exhaustive, i.e. as meaning "including, but not limited thereto."

Moreover, throughout the entirety of the document, unless the context otherwise requires, variations such as "include," "includes," "including," or the like shall be construed as not only including a group of singularity or plurality derived from ordinary verbs, but also not excluding the inclusion of any other group of singularity or plurality.

The invention according to the first feature provides a healthcare device that is provided with: a reception unit that receives first data related to at least one condition associated with a first user; a control unit that decides second data on the basis of at least the first data and healthcare data; and an output module that outputs the second data, the second data including first information and second information, the first information including at least one health condition of the first user, the second information including a degree of urgency with respect to each of the at least one health condition.

According to the invention of the first feature, the reception unit can receive a condition of a first user(patient) without an operator, thereby the healthcare device receiving a more objective condition of the patient without subjectivity of the operator.

Further, the control unit decides second data on the basis of first data related to at least one condition and healthcare data, then the output module outputting the decided second data. At that time, the second data includes: first information related to at least one health condition of the first user, and second information related to a degree of urgency with respect to each of such health condition. Therefore, the output module can output information related to the health condition of the patient and a degree of urgency of a medical treatment, with anamnesis more surely covered, by means of healthcare data.

Therefore, the invention according to the first feature provides a healthcare device: enabling the healthcare device to receive information that excludes subjectivity of an operator as much as possible; by adding such information and information covering more surely anamnesis from which the patient has suffered, grasping a health condition and a degree of urgency of the patient exemplified by a predicted disease, and a medical examination department of a specialized category related to a selected medical examination or the like thereby; and capable of selecting more correctly a necessary measure exemplified by a decision or the like of an inspection necessary for the patient, a medical treatment policy, a degree of urgency of a consultation, an order of a medical treatment for the patient, an order of an emergency transport, and a designated facility, on the basis of the health condition and the degree of urgency of the patient that are grasped.

The invention according to the second feature is the invention according to the first feature, the invention providing the healthcare device, wherein the at least one health condition includes a possibility of injury, a possibility of disability, and/or a possibility of illness.

According to the second feature, the healthcare device can output not only a health condition that can be surely diagnosed but also a possible health condition, with respect to the first user(patient). This allows provision of the healthcare device that can decide more correctly the health condition and the degree of urgency of the patient related to a selection or the like of a necessary measure, on the basis of the health condition and the degree of urgency of the patient.

The invention according to the third feature is the invention according to the first or second feature, the invention providing the healthcare device, wherein the healthcare data is data that the reception unit receives from outside of the healthcare device.

According to the invention of the third feature, the healthcare device can be configured without a large storage device or the like storing healthcare data, and structured small and portable. A portable healthcare device can receive information that is more objective and covers the anamnesis more surely, even at a place far from a medical institution or the like in which a large healthcare device can be arranged, for example, an emergency medical care site, a nursing facility, etc. Further, as a storage device or the like storing healthcare data can be configured without the portability of the healthcare device, a storage device or the like storing more healthcare data can be constructed. Then, the healthcare device can receive information that is more objective and covers the anamnesis more surely.

The invention according to the fourth feature is the invention according to any of the first to third features, the invention providing the healthcare device, wherein the reception unit receives the first data when a second user for whom a predetermined registration has been made succeeds in an authentication of a user.

According to the invention of the fourth feature, a second user for whom a predetermined registration has been made is a user of the healthcare device, thereby it being not possible that important personal information of the condition of the first user(patient) is spread to a third party for whom no registration has been made. Therefore, the patient can provide objective information excluding the subjectivity of the operator without worries about a leakage of personal information.

The invention according to the fifth feature is the invention according to the fourth feature, the invention providing the healthcare device, wherein the authentication includes face authentication, fingerprint authentication, iris authentication, voiceprint authentication, vein authentication, card authentication, or a combination thereof.

According to the invention of the fifth feature, during the authentication of the second user as a user, a reliable authentication is performed by means of various biological information or a predetermined card, thereby the first user(patient) providing more objective information without less worries about a leakage of personal information.

The invention according to the sixth feature is the invention according to the fourth or fifth feature, the invention providing the healthcare device, wherein the first user is the same as the second user.

According to the invention of the sixth feature, the first user(patient) himself/herself performs the authentication, thereby the healthcare device associating the patient with the healthcare data accurately, on the basis of a discrimination of the patient through the authentication. This prevents a mistake of the healthcare data of the patient with healthcare data of another patient. Therefore, the healthcare device can receive information that is more objective and covers the anamnesis more surely.

The invention according to the seventh feature is the invention according to any of the first to sixth features, the invention providing the healthcare device, wherein the healthcare data includes perinatal period healthcare data related to perinatal period healthcare associated with the first user, and/or infant healthcare data associated with an infant who is a child of the first user.

In a perinatal period, which is before or after birth, a health condition of a mother affects a health condition of a child, and vice versa. Therefore, it is important to decide the health conditions of both the mother and the child, and to perform a decision or the like of an inspection necessary for the patient, a medical treatment policy, a degree of urgency of a consultation, an order of a medical treatment for the patient, an order of an emergency transport, and a designated facility, on the basis of the decided health condition.

According to the invention of the seventh feature, the healthcare device can receive information that includes healthcare data related to perinatal period healthcare associated with the first user(patient), is more objective, and covers the anamnesis more surely. Moreover, by means of healthcare data associated with an infant who is a child of the patient, the healthcare device can not only decide more correctly a health condition and a degree of urgency related to the child, but also decide more correctly a health condition and a degree of urgency related to the patient who is the mother affected by the health condition of the child. Therefore, the healthcare device can decide more correctly the health condition and the degree of urgency of the patient related to a selection or the like of a necessary measure on the basis of the health condition and the degree of urgency of the patient more appropriately, compared to the case of using only the health condition and the healthcare data of the patient.

The invention according to the eighth feature is the invention according to any of the first to seventh features, the invention further provided with an input command unit that commands an input of third data on the basis of the first data, the third data including: an additional condition associated with the first user; and/or the healthcare data of addition, the reception unit receiving the third data that is input according to the command, the control unit deciding the second data on the basis of the third data in addition to the first data and the healthcare data.

According to the invention of the eighth feature, by commanding the first user(patient) to input an answer to a necessary question and/or necessary healthcare data on the basis of the first data, the healthcare device can receive information that is more objective and covers the anamnesis more surely. Therefore, it is possible to provide the healthcare device that can decide more correctly the health condition and the degree of urgency of the patient related to a selection or the like of a necessary measure, on the basis of the health condition and the degree of urgency of the patient.

The invention according to the ninth feature is the invention according to the eighth feature, the invention providing the healthcare device further provided with: a generation unit that generates a health condition candidate group comprising one or more health condition candidates that are possible as health conditions of the first user, on the basis of the first data and the healthcare data; a removal unit that removes zero or more of the health condition candidates with a relatively low relevance to the health condition of the first user from one or more of the health condition candidates included in the health condition candidate group on the basis of the first data, the healthcare data, and the third data; and a determination unit that determines the presence/absence of one or more confirmable health conditions that can be defined as the health conditions of the first user on the basis of the first data, the healthcare data, and the third data, the control unit: deciding the second data on the basis of the health condition candidate group removed by the removal unit, the healthcare data, and the one or more confirmable health conditions when the determination unit determines the presence of the confirmable health conditions; and deciding the second data on the basis of the health condition candidate group removed by the removal unit, and the healthcare data when the determination unit determines the absence of the confirmable health conditions.

In the grasp of the degree of urgency, it is important to presume a severe disease even if it unlikely occurs, and a leakage of a possible disease is inadmissible.

According to the ninth feature, the generation unit can generate a health condition candidate group including possible diseases without omission, on the basis of the condition and the healthcare data of the first user(patient). Then, by means of third data that is obtained from an input of an answer to a necessary question commanded on the basis of a condition of the patient and from the condition of the patient, and of the healthcare data covering more surely the anamnesis of the patient, a removal unit removes a disease predicted to have a relatively low relevance to a disease or the like of the first user. Further, the determination unit determines the presence/absence of confirmable health conditions that can be defined as the disease or the like of the first user by means of the aforementioned third data and the healthcare data.

Then, when the determination unit finds a confirmable disease as the disease of the patient, the control unit can decide the second data by means of this confirmable disease. Thus, it is possible to grasp the disease of the patient quickly. Additionally, the control unit decides the second data not only by a confirmable disease but also by a health condition candidate group removed by the removal unit, and healthcare data. Therefore, it is possible to avoid occurring a leakage in a presumed severe disease even if it unlikely occurs to grasp a degree of urgency accurately.

On the other hand, when the determination unit doesn't find the confirmable disease as the disease of the patient, the control unit decides the second data by the health condition candidate group removed by the removal unit, and the healthcare data. Therefore, it is possible to make the most use of present information, to minimize the risk of a leakage in the presumed severe disease even if it unlikely occurs, and to predict the degree of urgency as accurately as possible.

Therefore, the invention according to the ninth feature provides the healthcare device, regardless of whether the confirmable disease as the disease of the patient is found or not, that can decide more correctly the health condition and the degree of urgency of the patient related to a selection or the like of a necessary measure, on the basis of the health condition and the degree of urgency of the patient.

The invention according to the tenth feature is the invention according to the eighth or ninth feature, the invention providing the healthcare device further provided with a machine learning unit that causes a neural network to perform machine learning related to the health condition and the degree of urgency of the health condition by means of the first data and the third data, the control unit deciding the second data on the basis of the neural network in addition to the first data, the healthcare data, and the third data.

According to the invention of the tenth feature, the healthcare device can perform machine learning of a neural network on the basis of the condition of the patient, the healthcare data, and/or the question that are stored, and can decide the health condition of the patient that is presumed from the condition of the patient, the healthcare data, and the question. Further, on the basis of the machine learning, the healthcare device can find a new relevance: between the condition of the patient, the healthcare data, and the question; and a disease, and can increase the accuracy of deciding the disease and the degree of urgency. By using machine learning, without spending labor and time for developers or the like of the healthcare device to refer to enormous first data and third data in order to search a relevance between these data and a disease, the healthcare device can decide which disease and degree of urgency exists. This means that it is possible to provide the healthcare device that can decide more correctly the health condition and the degree of urgency of the patient related to a selection or the like of a necessary measure, on the basis of the health condition and the degree of urgency of the patient.

The invention according to the eleventh feature is the invention according to any of the first to tenth features, the invention providing the healthcare device, wherein the second data further includes information related to: a healthcare measure to be performed to the first user; and/or healthcare advice for the first user.

According to the eleventh feature, the healthcare device can provide a healthcare measure and/or healthcare advice that are necessary for the first user(patient). Therefore, it is possible to provide the healthcare device that can decide more correctly the health condition and the degree of urgency of the patient related to a selection or the like of a necessary measure, on the basis of the health condition and the degree of urgency of the patient.

The invention according to the twelfth feature is the invention according to any of the first to eleventh features, the invention providing the healthcare device, wherein the healthcare data includes: gene data of the first user; data related to mind of the first user; and/or data related to a living environment of the first user.

According to the invention of the twelfth feature, the healthcare device considers not only the condition of the first user(patient) but also gene data, a mental condition, and/or a living environment of the patient to decide which disease and degree of urgency exists more accurately. Further, on the basis of a combination of two or more information selected from the condition of the patient, the healthcare data of the patient, the gene data, the mental condition, and/or the living environment of the patient, the healthcare device can decide more correctly the health condition and the degree of urgency of the patient related to an order of the medical treatment for the patient, an order of the emergency transport, a designated facility, etc., compared to a decision based on each factor. Therefore, it is possible to provide the healthcare device that can decide more correctly the health condition and the degree of urgency of the patient related to a selection or the like of a necessary measure, on the basis of the health condition and the degree of urgency of the patient.

The invention according to the thirteenth feature provides a system that is configured to be provided with: the healthcare device according to any of the first to twelfth features; and an authentication module that is provided separate from the healthcare device and can authenticate that a second user for whom a predetermined registration has been made is a user, the reception unit receiving the first data when the authentication module authenticates the user to be the second user.

According to the invention of the thirteenth feature, a second user for whom a predetermined registration has been made is a user of the healthcare device, thereby important personal information of the condition of the first user(patient) not spread to a third party for whom no registration has been made. Therefore, the patient can provide objective information excluding subjectivity of an operator without worries about a leakage of personal information.

Further, according to the invention of the thirteenth feature, an authentication module that can authenticate that the second user for whom the predetermined registration has been made is the user is configured separate from the healthcare device. Therefore, the healthcare device can be configured without the authentication module, and structured small and portable. A portable healthcare device can receive information that is more objective and covers the anamnesis more surely, even at a place far from a medical institution or the like in which a large healthcare device can be arranged, for example, an emergency medical care site, a nursing facility, etc. Therefore, it is possible to provide the system for medical use that can perform a more accurate selection or the like of a necessary measure, on the basis of the health condition and the degree of urgency of the patient.

The invention according to the fourteenth feature provides a system that is configured to include: the healthcare device according to any of the first to twelfth features; and a server that is provided separate from the healthcare device, the server having a database that stores the healthcare data, the reception unit receiving the healthcare data from the database.

According to the invention of the fourteenth feature, a server can be configured separate from the healthcare device, thereby the healthcare device being configured without a server, and structured small and portable. A portable healthcare device can receive information that is more objective and covers the anamnesis more surely, even at a place far from a medical institution or the like in which a large healthcare device can be arranged, for example, an emergency medical care site, a nursing facility, etc. Further, a server can be configured without taking any account of portability of the healthcare device, thereby a database that stores more data being constructed. Then, the healthcare device can receive information that is more objective and covers the anamnesis more surely.

The invention according to the fifteenth feature is the invention according to the fourteenth feature, the invention providing the system, wherein the server further has an authentication module that can authenticate that a second user for whom a predetermined registration has been made is a user.

According to the invention of the fifteenth feature, an authentication module can be configured integrally with the server, thereby the authentication module authenticating only a second user (for example, a medical worker) who is present at a medical institution in which the server is arranged. This means that the authentication of the second user is more reliable. A reliable authentication is performed, thereby the first user(patient) providing the condition of the patient and/or the healthcare data accurately without less worries about a leakage of personal information. Therefore, the healthcare device can receive information that is more objective and covers the anamnesis more surely.

The invention according to the sixteenth feature provides the system that is configured to include: the healthcare device according to any of the first to twelfth features; and a sensor that is provided separate from the healthcare device, the sensor acquiring at least one image data of the first user and outputting the at least one acquired image data as a part or all of the first data on the reception unit.

According to the sixteenth feature, a sensor can be configured separate from the healthcare device, thereby the healthcare device being configured without a sensor, and structured small and portable. This assures that the healthcare device is portable and receives more objective information, even at a place far from a medical institution or the like in which a large healthcare device can be arranged, for example, an emergency medical care site, a nursing facility, etc. Further, a sensor can be configured without taking any account of portability of the healthcare device, thereby the system being constructed so as to include a large sensor that can acquire more information. Therefore, the healthcare device can receive more information that is more objective and covers the anamnesis more surely.

Further, according to the sixteenth feature, the healthcare device can receive image data of the patient that is objective data excluding subjectivity of an operator. Therefore, it is possible to provide the system that can decide more correctly the health condition and the degree of urgency related to a selection or the like of a necessary measure, on the basis of the health condition and the degree of urgency of the patient.

The invention according to the seventeenth feature is the invention according to the sixteenth feature, the invention providing the system, wherein there is a plurality of the image data, the sensor acquiring these plurality of the image data at different timing.

According to the invention of the seventeenth feature, on deciding the second data including information related to the health condition and the degree of urgency on the basis of: the first data including a plurality of image data; and the healthcare data, the control unit can perform the grasp of a pace of a symptom, and the diagnosis of a disease that a symptom changes depending on time zones of a day, or the like, by comparing each of a plurality of image data of the first user(patient) acquired at different timing. Therefore, it is possible to provide the system that can decide the health condition and the degree of urgency of the patient related to a decision or the like of an inspection necessary for the patient, a medical treatment policy, a degree of urgency of a consultation, an order of a medical treatment for the patient, an order of an emergency transport, and a designated facility.

The invention according to the eighteenth feature is the invention according to the seventeenth feature, the invention providing the system, wherein the control unit of the healthcare device is further provided with a mental condition determination unit that determines a mental condition of the first user on the basis of the plurality of the image data, the control unit deciding the second data on the basis of the first data and the mental condition.

According to the invention of the eighteenth feature, on deciding the second data including information related to the health condition and the degree of urgency, the control unit of the healthcare device can determine a mental condition by means of the image data of the first user(patient) acquired at different timing to decide a health condition including not only a physical disease but also a mental disease of the patient. Further, on deciding the second data, such control unit considers the synergistic effect of the physical disease and the mental condition to decide the degree of urgency of the health condition. Therefore, it is possible to provide the system that can decide the health condition and the degree of urgency of the patient related to a decision or the like of an inspection necessary for the patient, a medical treatment policy, a degree of urgency of a consultation, an order of a medical treatment for the patient, an order of an emergency transport, and a designated facility.

The invention according to the nineteenth feature is the invention according to any of the sixteenth to eighteenth features, the invention providing the system, wherein one or more image data of the at least one image data includes thermal image data.

According to the invention of the nineteenth feature, on deciding the second data including information related to the health condition and the degree of urgency, the control unit of the healthcare device can measure a surface temperature of the skin of the first user(patient) by means of the first data including thermal image data to decide the second data on the basis of various objective information of the patient such as peripheral circulatory insufficiency of the patient, the presence/absence of right and left difference of a body temperature caused by a paralysis, a dermatosis, and/or a temperature difference from a peripheral part such as hemangioma, etc. Therefore, according to the invention of the nineteenth feature, it is possible to provide the system that can provide information that is more objective and covers the anamnesis more surely.

The invention according to the twentieth feature and the invention according to the twenty-first feature are the inventions whose category is different from the invention according to the first feature.

### EFFECTS OF THE INVENTION

According to the present invention, it is possible to provide a healthcare device, a system, and a method that enables the healthcare device to receive information excluding subjectivity of an operator as much as possible, adds such information and information covering more surely anamnesis from which a patient has suffered to grasp a health condition and a degree of urgency of the patient exemplified by a predicted disease, thereby a medical examination department of a specialized category related to a selected medical examination or the like, and selects more correctly a necessary measure exemplified by a decision or the like of an inspection necessary for the patient, a medical treatment policy, a degree of urgency of a consultation, an order of a medical treatment for the patient, an order of an emergency transport, a designated facility, on the basis of the health condition and the degree of urgency of the patient that are grasped.

Description of various embodiments is given only by examples, with a reference to the associated drawings, in the following examples.

### BRIEF DESCRIPTION OF DRAWINGS

Fig. 1 is a block diagram illustrating a system 1 according to some embodiments of the present disclosure.
Fig. 2 is a flowchart illustrating a system 1 according to some embodiments of the present disclosure.
Fig. 3 is a block diagram illustrating an example of a system 1001 of the second embodiment of the present invention.
Fig. 4 is a view illustrating an example of a healthcare data table 1221.
Fig. 5 is a view illustrating an example of a health condition table 1222.
Fig. 6 is a view illustrating an example of an input command table 1223.
Fig. 7 is a view illustrating an example of a candidate table 1224.
Fig. 8 is a flowchart illustrating an example of the flow of a triage process by means of a system 1001 according to the present embodiment.
Fig. 9 is a view followed by Fig. 8.

### DETAILED DESCRIPTION

In the following, description is given of an example of a preferred aspect for carrying out the present invention with reference to the figures. It is to be noted that this is merely an example and the technical scope of the present invention is not limited thereto.

Unless otherwise explicitly defined, any other technological or scientific terms used in the description have the same meaning as the meaning understood generally by a person skilled in the art belonging to the theme of the description.

### [[First Embodiment]]

In the following, description is given of the first embodiment of the present invention.

### [System 1]

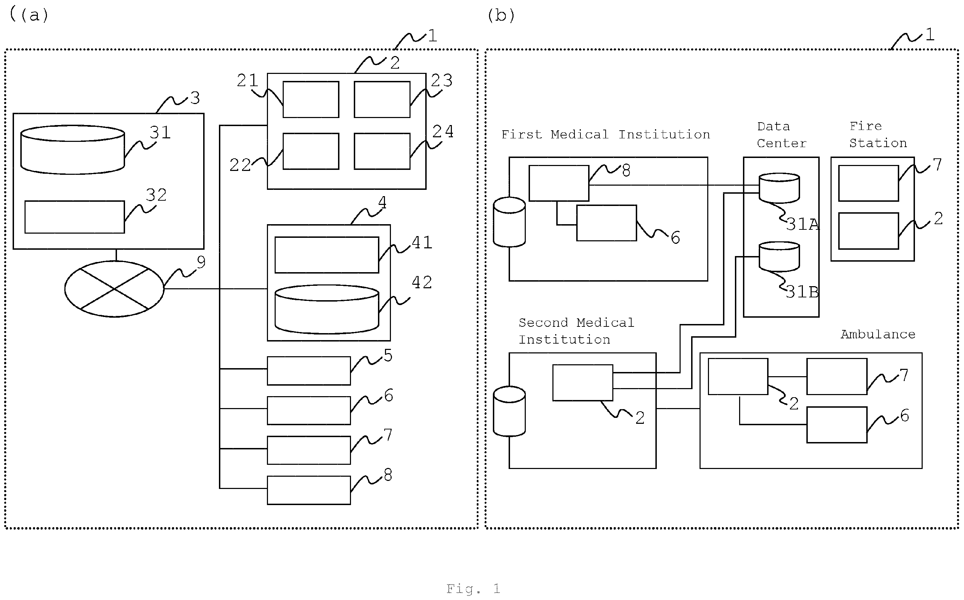

With reference to Figs. 1 (Fig. 1A and Fig. 1B) and 2, description is given of a non-limiting example of a system 1. As shown in Fig. 1A, the system 1 may be provided with a healthcare device 2 and an authentication device 4. Further, the system 1 may be used for medical use such as medical diagnosis, medical checkup, etc.

The system 1 may be further provided with a server 3, a first device 5, a second device 6, a third device 7, and a fourth device 8. At least one selected from the healthcare device 2, the server 3, the authentication device 4, the first device 5, the second device 6, the third device 7, and the fourth device 8 may include a reception unit configured to receive data, a control unit configured to process data from the reception unit, and a transmission unit set to transmit data processed under control of the control unit. The first device 5, the second device 6, the third device 7, and the fourth device 8 may be provided separate from the system 1.

### [Healthcare Device 2]

Firstly, description is given of the healthcare device 2. As shown in Fig. 1A, the healthcare device 2 includes a reception unit 21, a control unit 22, an output module 23, and a transmission unit 24. In some embodiments, the healthcare device 2 may be used for outputting first information and second information. The healthcare device 2 is a personal computer, a cell phone, or a tablet, but not limited thereto. The healthcare device 2 may be a device configured to communicate with other devices (for example, the server 3, the authentication device 4, the first device 5, and/or the second device 6).

The reception unit 21 is configured to receive data from other devices, and a physical keyboard displayed on a display (not shown) of the healthcare device 2 or a software keyboard, a microphone (not shown) recognizing voice, or a combination thereof may be configured. Received data may be provided to the control unit 22. The control unit 22 may be configured to process data provided from the reception unit 21.

The output module 23 may output data on the basis of data output from the control unit 22 and/or data output from an authentication module 41, or may output via, for example, a display or a speaker. On the basis of the data output from the control unit 22 or the data output from the authentication module 41, the transmission unit 24 may transmit data to other devices (i.e., the server 3, the authentication device 4, the first device 5, and/or the second device 6).

It should be understood that the reception unit 21 may receive data (for example, first data) from other devices when a second user succeeds in an authentication. The first data may be stored outside of the healthcare device 2. In this regard, in order to show that an authentication against the second user is succeeded successfully, the reception unit 21 may receive data from the authentication device 4 before receiving healthcare data from other devices.

Data output from the output module 23 and transmitted from the transmission unit 24 include at least one health condition or at least one biological parameter. The data output from the output module 23 and transmitted from the transmission unit 24 include each degree of urgency against the at least one health condition or the at least one biological parameter. In the following, the at least one health condition and/or the at least one biological parameter may be referred as first information, and each degree of urgency against the at least one health condition and/or the at least one biological parameter may be referred as second information.

A condition and/or a biological parameter may include, for example, health, injury, diabetes, illness such as heart disease, autoimmunity, a cancer, a tumor, schizophrenia, pneumonia, halitosis, alcohol intake, organ psychological trauma, leukemia, cutaneous lymphoma, hepatoma, a disability such as neuropathy, another health condition and/or biological parameter, but not limited thereto.

In order to update information related to the first user, the transmission unit 24 may transmit various data related to the first user stored in the healthcare device 2 to the server 3 in every determined period.

### [Server 3]

Secondly, description is given of the server 3. As shown in Fig. 1A, the server 3 may be configured so as to communicate with the healthcare device 2, the authentication device 4, the first device 5, the second device 6, the third device 7, and the fourth device 8. Communication is performed by means of, for example, wireless communication, wire communication, etc. A wireless protocol or a standard may include at least one of, for example, Wireless Fidelity (Wi-Fi)^{®} such as IEEE 802.11 a/b/g/n/ax/ba/EHT or the like, Bluetooth^{®}, near field communication (NFC), radio frequency identifier (RFID), and mobile communication (for example, Long term Evolution (LTE), LTE-Advanced (LTE-A), and 5th Generation (5G). A wire communication module may include at least one of Universal Serial Bus (USB), and High-Multimedia Definition Interface (HDMI^{®}), Recommended Standard 232 (RS-232).

The server 3 may include a database 31 and a communication module 32. The database 31 includes healthcare data of a user, is updated every time the server 3 communicates with at least one of the healthcare device 2, the authentication device 4, and/or the first device 5, and can update the second device 6, the third device 7, and the fourth device 8. Therefore, the database 31 can store the newest information. The communication module 32 may transmit data stored in the database 31 to another device/apparatus, and may store data transmitted from another device (for example, the healthcare device 2, the authentication device 4, the first device 5, and/or the second device 6) in the database 31. For example, the server 3 may transmit the data stored in the database 31 according to a request of the healthcare device 2.

The term of "a user" includes i)a patient, ii)a worker for a specific company or at a medical institution such as a hospital, and iii)another personnel who is allowed for an access to a system, but not limited thereto. A user inspected by means of the system 1 may be called as a first user, and, compared to the first user, a user using the system 1 may be called as a second user. The first user may be a patient and the second user may be a staff or another personnel.

The server 3 may be configured to transmit data in the database 31 related to personal data (first data) of the first user, according to a request of the second user. Examples of personal data are mentioned later.

### [First Device 5]

In the next, prior to description of the authentication device 4, description is given of the first device 5. As shown in Fig. 1A, the first device 5 may include a sensor (not shown). The first device 5 may be configured to include a camera arranged in a medical institution such as a hospital or the like, or may be configured to include another device. A sensor may acquire at least one image of at least one part of the body of the first user, or may be an image acquisition sensor. At least one acquired image may be a still image or a frame of a video. As the sensor acquires at least one image, the sensor may transmit data to the server 3 and/or the healthcare device 2. The sensor may further analyze a characteristic of at least one acquired image data.

In the case that the sensor acquires a plurality of images, the sensor analyzes the vibration degree of the first user (i.e. a change of positions of a detected part included in a plurality of images). The vibration degree may be associated with an extent of move of a predetermined period, a person, an individual, or a body part of a subject (for example, spasms of an eye, a raise of an eyebrow, a dilation of pupils of eyes, a tension of facial muscles, etc.). The vibration degree may be decided on the basis of a comparison with a plurality of images acquired at different timing. After the vibration degree being established or decided, the sensor can be operated in order to decide a condition of the first user on the basis of the vibration degree. It should be understood that a great vibration degree associated with a single body part or a plurality of body parts may show an emotional condition, a physical condition, or a mental condition. Various conditions may be used in order to derive an index of aggression, tension, normality, rest/rest, or fatigue, related to a facial expression of the first user.

It should be understood that image data may be acquired at different timing. It should be understood that the first device 5 may be provided on the healthcare device 2 in order to acquire at least one image. It should be understood that the sensor may further decide a condition on the basis of an image acquired from the sensor.

It should be understood that at least one image data may be acquired by a still camera, a video camera, or a device with a photographing and/or video-recording function. The still camera or the video camera may be a surveillance camera arranged in a firstly decided area (for example, a street, or a platform of a station), or a detection camera arranged on a secondly decided area (for example, an immigration control area in an airport).

It should be understood that the first device 5 may decide the condition of the first user, not by acquiring at least one image, but by acquiring a thermal image of the first user on the basis of an acquired thermal image. In the case that the first device 5 acquires the thermal image of the first user, the sensor may be an infrared sensor for acquiring a thermal image.

### [Authentication Device 4]

Then, description is given of the authentication device 4. As shown in Fig. 1A, the authentication device 4 may be provided with an authentication module 41 and a database 42, and may be used for the purpose that data stored in the second device 6 is safely used.

The authentication module 41 can be configured to communicate with a device including the second device 6 or the like, such as a personal computer, a mobile device, a tablet, etc. The authentication module 41 may be an RFID reader, a fingerprint reader, or a combination thereof.

The database 42 can include a list of an approved person (a second user) who is allowed to refer to data stored in the second device 6 therein. The approved person may include not only a person who possesses the second device 6 but also at least one person who belongs to an approved institution (for example, a medical institution). Authentication data may be a list of an approved person (for example, a patient) and/or institution (for example, a medical institution). Personal data and authentication data may be included in an RFID tag so that the authentication module 41 reads personal data and authentication data included in the second device 6. It should be understood that the healthcare device 2 may receive information by scanning a quick response (QR) code that shows information on the second device 6.

As the authentication module 41 acquires data stored in the second device 6, the authentication module 41 may be configured to authenticate if a second user is an approved person or not. Especially, the authentication module 41 may require the second user to input data distinguishing second data such as a user account, a password, etc. The authentication module 41 may perform an authentication by comparing input information stored in the database 42 to data.

The authentication module 41 may transmit data to the healthcare device 2 and/or the server 3. In the case that the second user is approved, the authentication module 41 may transmit data included in the database 42 to the server 3 so that the server 3 stores data in the database 31. In the case that the second user is approved, the authentication module 41 may transmit data included in the database 42 to the healthcare device 2 so that the control unit 22 processes data received from the authentication module 41.

The second user is firstly required to be authenticated by means of the authentication device 4. On authentication, the second user may input personal data such as a chief complaint, i.e. a main symptom that a patient complains to a doctor, or the like, on the healthcare device 2 via the reception unit 21. The second user inputs a medical problem, then the output module 23 may output first information and second information under control of the control unit 22.

Instead of using the authentication device 4, the healthcare device 2 may have functions of the authentication module 41 acquiring information on the second device 6, and of the database 42. Therefore, it should be understood that the device 4 for the authentication of the second user doesn't have to be used for authentication.

In the case that the authentication of the second user is performed, it should be understood that other authentication methods may be used. Other authentication includes, for example, (i) face authentication by camera (not shown), (ii) password authentication by password input, (iii) fingerprint authentication by means of fingerprint reader (not shown), (iv) iris authentication by means of IRIS camera (not shown), (v) voiceprint authentication, (vi) vein authentication, and (vii) authentication by means of a combination thereof, etc.

It should be understood that the healthcare device 2 and/or the authentication device 4 may include a part of a device and/or an application according to at least one of authentication methods to be used. For example, the healthcare device 2 and/or the authentication device 4 may include a camera performing face authentication.

It should be understood that the database 31 may store the same data as the authentication device 4. Hence, the server 3 may authenticate the second user.

### [Second Device 6]

The second device 6 may be configured as a personal computer, a mobile device, a tablet, an IC card, or the like, or may include personal data in the first user. The second device 6 is configured as an identification card issued by a hospital to get a medical checkup. Alternatively, the second device 6 may be configured as a credit card, a debit card, a loyalty card, a prepaid transportation card, a student ID card, or a combination thereof. The second device 6 may have personal data in the first user.

Personal data includes a symptom, a physical feature, and healthcare data of the first user, but not limited thereto, and may be associated with a diagnosis by a medical worker such as a doctor or the like, a consultation, a medical checkup, a physiological inspection, prescription, a pathological inspection, a radiographic inspection, a vital sign, a laboratory inspection, family history, height, weight, a blood pressure, a blood sugar level, a basal body temperature, history of drug in the past, a drug response data including a pharmaceutical identity, an allergy, age, gender, race, an insurance company, a place of residence, contact information, an identification number, history of places of purchased merchandises, a web interaction, email communications, request history, payment history, a typical billed amount, time of a day, a period of online interactions, a number of online interactions, family status, an occupation, a transaction, a phone number, age, gender, race, blood type, an insurance company, an allergy, medical history in the past, family history, society history, religion, an employer, a guarantor, an address, contact information, and/or a condition code of a patient, etc.

It should be understood that information stored in the second device 6 may be updated every time when a user uses the second device 6 at a specific place such as a medical institution or the like. In this way, the system 1 can provide a medical checkup and/or information indicating priority of disease of a user, on the basis of the newest information.

It should be understood the second device 6 may be configured as a wearable device (wristwatch type, glasses type, ring type, shoes type, flash type, pendant type, clothing type, a pacemaker, an IC chip (paste-type, embedded type), or the like). The authentication device 4 may use a wearable device to receive data stored in the wearable device. The stored data may include a body temperature, a blood pressure, a pulse, an electrocardiogram, a fetal heart race, a blood sugar level, respiration, oxygen saturation, jaundice, skin color, a number of steps, weight, a sleeping time, calories, sense of balancing, a posture, a falling, mastication, a taste, sense of hearing, a perception, a temperature, an atmosphere pressure, ultraviolet radiation, or the like, but not limited thereto.

### [Third Device 7]

The third device 7 may be configured as a personal computer, a mobile device, a tablet, an IC card, or the like, may include personal data in the second user, or may be used for an authentication of the second user.

### [Fourth Device 8]

The fourth device 8 can be used in the case that a new healthcare card of the second device 6 or the like is issued at a specific place such as a hospital. Especially, the fourth device 8 may embed patient data in the new healthcare card.

### [Examples of flowchart]

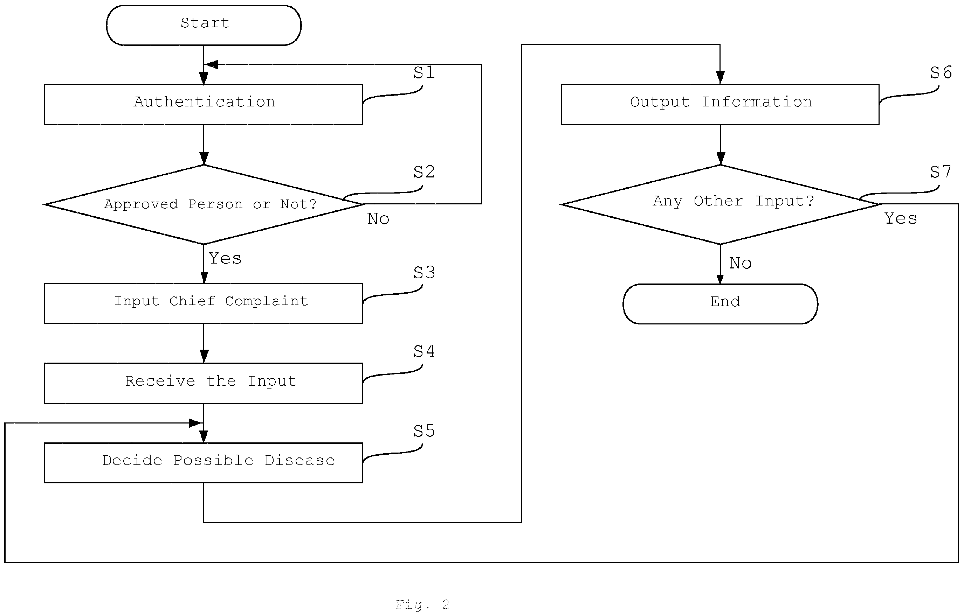

With reference to Fig. 2, description is given of a detailed flowchart of the present disclosure. As shown in Fig. 2, there is a processing including S11 to S17, which is performed by the system 1. In the following, the authentication module 41 and the database 42 may be provided in the healthcare device 2. In this case, the healthcare device 2 may perform the processing of S11 and S12.

In S11, the healthcare device 2 may start an authentication of the second user, which determines if the second user is an approved person or not. In S12, in the case that the healthcare device 2 determines if the second user is an authenticated person or not (i.e. the determination result in S12 is yes), the following processing may be shifted to the next step (i.e. S13). Received data includes data stored in the second device 6. It should be understood that an authentication process such as S11, S12, or the like, may be omitted.

In S13, the second user may operate the healthcare device 2 and input a medical issue. A medical issue may include a headache, a stomachache, a backache, anemia, constipation, and the current condition of the first user, but not limited thereto. On the basis of the medical issue, the healthcare device 2 may decide first data and second data. This healthcare device 2 further decides conditions that may be categorized as (i) further condition (other diseases that the extension of the current condition may occur), and/or (ii) not-available condition (other diseases that the current condition avoids). In S14, the healthcare device 2 may receive data stored in the second device 6 in S15, and the healthcare device 2 may decide the first data and the second data on the basis of data from the authentication device 4 and of Artificial Intelligence (AI).

In S16, the output module 23 may output the first information (i.e. at least one possible disease of the first user) and the second information (i.e. a degree of urgency against each of the at least one possible disease). In S16, the output module 23 may output information indicating what a user should do. The information what the user should do includes, (i) a necessary inspection and medical treatment, (ii) a hospital that the user must visit to see a doctor, (iii) a methodology for preventing diseases, and/or (iv) a meal that the user should have, a recipe of a meal, a delivery food, carbohydrate-restricting food, an allergy, any other contraindicated food, or the like, but not limited thereto. What the user should do results in preventing a progression or a deterioration of the condition of the first user. Therefore, a preferable action for the first user may be specified. In S17, the healthcare device 2 may decide if any data is provided from other devices or not. In the case that the data is provided from other devices in S17, the processing may be shifted to S15. In the case that the data provided from other devices is not provided from other devices in S17, the processing may be ended.

### [Other examples of System 1]

Fig. 1B is a block diagram illustrating the system 1 according to some embodiments of the present disclosure. As shown in Fig. 1B, the second device 6 and the fourth device 8 can be used at a first medical institution, the healthcare device 2 may be provided at a second medical institution, and the healthcare device 2, the second device 6, and the third device 7 are arranged in an ambulance. Card information that can store information related to a medical institution and a worker at a medical institution, and a database 31A that can store a database 31B are provided to a data center, the healthcare device 2 and the third device 7 being provided at a fire station.

As described above, the fourth device 8 may be used when a new healthcare card of the second device 6 or the like is issued at a first medical institution. As the healthcare card is issued, data stored in the healthcare card may be transmitted to the data center. Especially, the database 31A in the data center may store data stored in the healthcare card. In the case that a specific accident happens on the first user, a worker in an ambulance can be authenticated by using the healthcare device 2 and the third device 3 to have an access to data stored in the second device 6. As the worker in the ambulance succeeds in an authentication, the processing of S15 to S17 shown in Fig. 2 is performed, thereby being able to decide at least one possible disease of the first user, and a degree of urgency against each of the at least one possible disease. As the at least one possible disease of the first user and the degree of urgency against each of the at least one possible disease are decided, first user details may be reported from the ambulance to the second medical institution. The worker at the second medical institution can be authenticated by using the healthcare device 2 and having an access to the database 31B to have an access to data stored in the second device 6. As the worker at the second medical institution succeeds in an authentication, the processing S15 to S17 shown in Fig. 2 may decide the at least one possible disease of the first user and the degree of urgency against each of the at least one possible disease. As for data such as the data stored in the second device 6 or the like, the at least one possible disease and/or the degree of urgency against each of the at least one possible disease may be transmitted from the healthcare device 2 to the database 31A.

It should be understood that, as the authentication device 4 authenticates the second user successfully, information included in the second device 6 may also be transmitted to the server 3 in S12.

In the case that the number of the at least one possible disease is 2 or more, it should be understood that the healthcare device 2 narrows down possible diseases by receiving more data from other devices.

Description is given of exemplary process S12 receiving information stored in the second device 6 as above, but additionally or alternatively, it should be understood that the healthcare device 2 may decide the at least one possible disease of the first user. Then, the healthcare device 2 may output the degree of urgency against each of the at least one possible disease on the basis of at least one data transmitted from the server 3 and the first device 5.

Description is given of exemplary process S12 receiving information stored in the second device 6 as above, but additionally or alternatively, it should be understood that the server 3 may decide the at least one possible disease and extent. The degree of urgency against each of the at least one possible disease is based on data from at least one of the following: the server 3, the first device 5, and the second device 6. Then, the healthcare device 2 may output the at least one possible disease and at least one possible degree of urgency against each of the at least one possible disease, on the basis of data from the server 3, the first device 5, and/or the second device 6.

### [Advantageous effects]

According to the present disclosure, the output module 23 is configured to output data indicating first information and second information, wherein the first information includes at least one possible disease and the second information includes a degree of urgency against each of diseases. Therefore, the second user can consider what measure needs to be taken in order to prevent a progression or a deterioration of the condition of a disease. Further, the user can decide what measure needs to be prioritized before taking a measure.

According to the present disclosure, the output module 23 is configured to output data indicating first information and second information, wherein the first information includes all of possible diseases derived from input data. Therefore, nevertheless one of the possible diseases is a severe disease that hardly occurs, the first user and/or the second user can consider a possibility of the severe disease to take a necessary measure.

According to the present disclosure, the healthcare device 2 is configured to receive data from other devices such as the server 3, the first device 5, and/or the second device 6, etc. Therefore, the system 1 can decide the first information and the second information from various aspects' perspective, thereby being able to acquire an accurate result.

According to the present disclosure, (i) personal data associated with data derived from a diagnosis by a medical worker such as a doctor or the like, and/or (ii) at least one image acquired from the first device 5 is construed to be initial information, thereby making it possible that second information decides terms correctly.

### [Possible derivations related to the first embodiment]

It should be understood that the first user may be the same as the second user (self-triage). This means that both the first user and the second user may be a common one patient.

It should be understood that image data acquired by the first device 5 may be uploaded to the server 3. It should be understood that data acquired from a card as the second device 6 may be previously uploaded to the server 3.

It should be understood that information in the database 31 and/or the second device 6 can put a weight coefficient, and the communication module 32 may decide at least one possible disease on the basis of the information and the weight coefficient. The weight coefficient may be decided according to the importance of an information coefficient.

The present disclosure (for example, the healthcare device 2, the server 3, the authentication device 4, the first device 5, the second device 6, the third device 7, and the fourth device 8) is configured by exemplary hardware, but may be provided by software cooperating with hardware.

Further, a functional block (not shown) used in hardware is typically implemented as an LSI device, which is an integrated circuit. The functional block may be formed as individual chips, or a part or all of the functional block may be integrated into a single chip.

### [[Second embodiment]]

The following explains the second embodiment of the present invention.

### [System 1001]

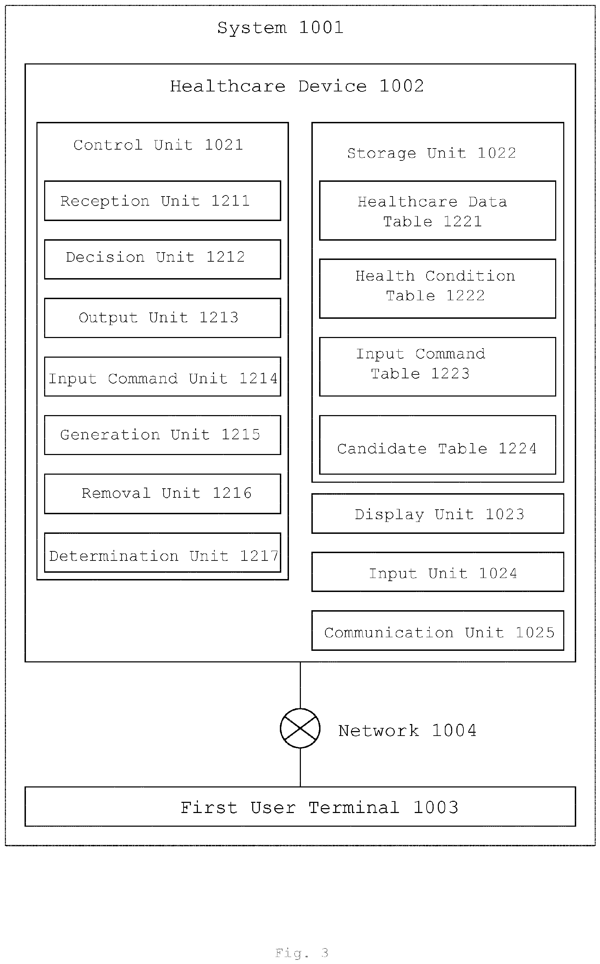

Fig. 3 is a block diagram illustrating an example of the configuration of a healthcare system 1001 (hereinafter simply referred to also as a system 1001) of the present embodiment.

The system 1001 is configured to include at least a healthcare device 1002. The system 1001 preferably includes one or several first user terminals 1003 and a network 1004 to be referred to later.

In the following, a patient whose health condition and degree of urgency are decided by means of the healthcare device 1002 is referred to also as a first user, and an operator operating the healthcare device 1002 and/or the first user terminal 1003 is referred to also as a second user. The second user includes, for example, the first user(patient), persons concerned of the patient including the family of the patient, a worker for a specific company or at a medical institution such as a hospital, and another personnel, etc.

The operator of the healthcare device 1002 is preferably a user who succeeds in an authentication that a second user for whom a predetermined registration has been made is a user. This prevents, as the second user for whom a predetermined registration has been made is a user of the healthcare device 1002, important personal information of the condition of the first user(patient) is spread to a third party for whom no registration has been made. Therefore, the patient can provide objective information excluding subjectivity of the operator, without worries about a leakage of personal information.

The authentication that the second user for whom a predetermined registration has been made is a user includes knowledge authentication by means of knowledge of the second user, possession authentication by means of a possession of the second user, biometric authentication by means of the biological information of the second user, or a combination thereof. The knowledge authentication includes, for example, password authentication that authenticates the second user by causing the second user to input a predetermined password associated with the second user, or the like. The possession authentication includes, for example, card authentication that authenticates the second user by means of a predetermined card that the second user possesses, an authentication that authenticates the second user by means of predetermined authentication information stored in a portable terminal that the second user possesses, etc. The biometric authentication includes, for example, face authentication, fingerprint authentication, iris authentication, voiceprint authentication, vein authentication, or an authentication by a combination thereof. On the authentication that the second user is a user, a reliable authentication by means of various biological information, a predetermined card, or the like, is performed, thereby the first user providing more objective information, without less worries about a leakage of personal information.

The second user may be the same as the first user. This assures that, as the first user(patient) performs an authentication by himself/herself, the healthcare device 1002 associates the patient with healthcare data correctly on the basis of the discrimination of the patient via authentication. This prevents a mistake of the healthcare data of the patient with healthcare data of another patient. Therefore, the healthcare device 1002 can receive information that is more objective and covers anamnesis more surely.

An authentication module (not shown) authenticating that the second user for whom a predetermined registration has been made is a user may be integrally configured with the healthcare device 1002, or may be separately configured from the healthcare device 1002. Preferably, the authentication module authenticating that the second user for whom a predetermined registration has been made can be separately configured from the healthcare device 1002. This assures that the healthcare device 1002 is configured without an authentication module, and structured small and portable. A portable healthcare device 1002 can receive information that is more objective and covers anamnesis more surely, even at a place far from a medical institution or the like in which a large healthcare device 1002 can be arranged, for example, an emergency medical care site, a nursing facility, etc. Therefore, it is possible to provide the system 1001 for medical use that can perform a more correct selection or the like of the health condition and the degree of urgency of the patient related to an order of a medical treatment for the patient, an order of an emergency transport, and a designated facility.

### [Healthcare Device 1002]

The healthcare device 1002 is configured to include, at least, a processing unit 1021, a storage unit 1022, a display unit 1023, an input unit 1024, and a communication unit 1025.

### [Processing Unit 1021]

The processing unit 1021 is provided with CPU (Central Processing Unit), RAM (Random Access Memory), and ROM (Read Only Memory), etc.

By reading a predetermined program and cooperating with the storage unit 1022, the display unit 1023, the input unit 1024, and/or the communication unit 1025 as required, the processing unit 1021 realizes a reception unit 1211, a decision unit 1212, an output unit 1213, an input command unit 1214, a generation unit 1215, a removal unit 1216, a determination unit 1217, or the like, which are factors of a software configuration of the healthcare device 1002.

### [Storage unit 1022]

The storage unit 1022 is a device that stores data and a file, the storage unit having a storage part of data such as a hard disk, a semiconductor memory, recording medium, a memory card, etc. The storage unit 1022 may have a mechanism that allows for connecting with a storage device such as NAS (Network Attached Storage), SAN (Storage Area Network), a cloud storage, a file server, and/or a distributed file system, or a storage system, via network.

The storage unit 1022 stores, at least, a control program performed in a microcomputer, a healthcare data table 1221, a health condition table 1222, an input command table 1223, and a candidate table 1224, etc.

### [Healthcare Data Table 1221]

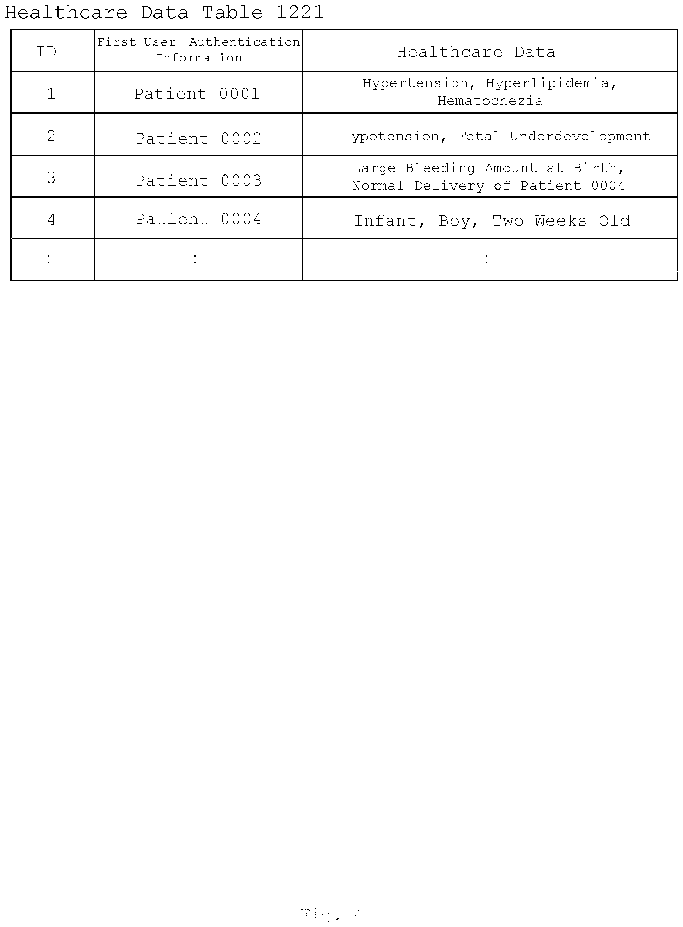

Fig. 4 is a view illustrating an example of a healthcare data table 1221. The healthcare data table 1221 shown in Fig. 4 stores information associated with an "ID" distinguishing healthcare data, "First User Authentication Information" storing the authentication information of the first user(patient) related to healthcare data, and "Healthcare Data" including healthcare data having at least information related to anamnesis from which the patient has suffered.

For example, in an example shown in Fig. 4, data associated with an ID "1", first user authentication information "Patient 0001", and healthcare data "Hypertension, Hyperlipidemia, Hematochezia" is stored. This enables deciding the health condition and the degree of urgency of the patient, by the facts that the first user(patient) who is authenticated by the authentication information "Patient 0001" has hypertension and hyperlipidemia, and that hematochezia, which means blood in stools, is found.

The inclusion of an ID facilitates acquiring and storing healthcare data. The inclusion of first user authentication information prevents mistaking the healthcare data of the patient for healthcare data of another patient. The inclusion of healthcare data including information related to the anamnesis from which the patient has suffered results in providing the healthcare device 1002 that can perform a grasp of the health condition of the patient and a more correct decision or the like of an order of a medical treatment policy, an order of an emergency transport, a designated facility, by means of information covering more surely the anamnesis from which the patient has suffered.

The healthcare data may include data for medical use related to the patient such as information included in a clinical record of the patient, the condition of the patient, a biological parameter of the patient and/or the result of a clinical examination related to the patient. This assures that the healthcare device 1002 considers not only the anamnesis or the like of the patient but also information included in a clinical record of the patient, the condition of the patient, a biological parameter of the patient and/or the result of a clinical examination related to the patient to decide accurately what disease the patient has and how urgent it is.

The healthcare data preferably includes gene data of the first user(patient), the mental condition, and/or the living environment. This assures that the healthcare device 1002 considers not only the condition of the patient but also the gene data of the first user(patient), the mental condition, and/or the living environment to decide accurately what disease the patient has and how urgent it is. Moreover, compared to a decision based on an individual factor, it is possible to provide a more correct decision of the health condition of the degree of urgency of the patient related to a decision or the like of a medical treatment policy of the patient, an order of an emergency transport, a designated facility, on the basis of a combination of two or more information selected from the condition of the patient, the healthcare data of the patient, the gene data of the patient, the mental condition, and/or the living environment. Therefore, it is possible to provide the healthcare device 1002 that can decide more correctly the health condition and the degree of urgency of the patient related to a decision or the like of a medical treatment policy of the patient, an order of an emergency transport, and a designated facility.

The healthcare data preferably includes perinatal period healthcare data related to perinatal period healthcare of the first user(patient), and/or infant healthcare data associated with an infant who is a child of the patient.

In a perinatal period, which is before or after birth, a health condition of a mother affects a health condition of a child, and vice versa. Therefore, it is important to decide the health conditions of both the mother and the child, and to perform a decision or the like of an order of a medical treatment for the patient, an order of an emergency transport, and a designated facility, on the basis of the decided health condition.

The healthcare data includes perinatal period healthcare data related to perinatal period healthcare of the patient, and/or infant healthcare data associated with an infant who is a child of the patient, thereby the healthcare device 1002 receiving information that includes the healthcare data related to perinatal period healthcare associated with the patient, is more objective, and covers the anamnesis more surely. Further, by using the healthcare data associated with an infant who is a child of the patient, the healthcare device 1002 can decide more correctly not only the health condition and the degree of urgency related to the child, but also the health condition and the degree of urgency related to the patient who is the mother affected by the health condition of the child. Therefore, the healthcare device 1002 can decide the health condition and the degree of urgency of the patient related to a decision or the like of an order of a medical treatment for the patient, an order of an emergency transport, and a designated facility more correctly and appropriately, compared to when using only the health condition and the healthcare data of the patient.

For example, in an example shown in Fig. 4, data associated with an ID "3", first user authentication information "Patient 0003", and healthcare data "Large Bleeding Amount at Birth, Normal Delivery of Patient 0004" is stored. This enables deciding the health condition and the degree of urgency of the patient by means of the perinatal healthcare data including the fact that the first user(patient) authenticated by the authentication information "Patient 0003" had a large bleeding amount at birth, and the normal delivery of Patient 0004. Further, in the example shown in Fig. 4, regarding Patient 0004 who is a child of the patient authenticated by the authentication information "Patient 0003", healthcare data "Infant, Boy, Two Weeks Old" is stored. This enables deciding more correctly not only the health condition and the degree of urgency related to the child, Patient 0004, but also the health condition and the degree of urgency related to the mother, Patient 0003, who is affected by the health condition of the child, Patient 0004.

Healthcare data may further include data related to a diagnosis by a medical worker such as a doctor or the like, a medical checkup at working space, a medical checkup at school, a physiological inspection, prescription, a pathological inspection, a radiographic inspection, vital sign, a laboratory inspection, family history, height, weight, blood pressure, blood sugar level, basal body temperature, history of drug in the past, drug response data including pharmaceutical identity, an allergy, age, gender, race, an insurance company, a place of residence, contact information, an identification number, history of places of purchased merchandises, a web interaction, email communications, request history, payment history, a typical billed amount, time of a day, a period of online interactions, a number of online interactions, family status, an occupation, a transaction, a phone number, age, gender, race, blood type, an insurance company, an allergy, medical history in the past, family history, society history, religion, an employer, a guarantor, an address, contact information, and/or a condition code of a patient. The inclusion of such data enables the healthcare device 1002 to decide the health condition and the degree of urgency of a medical treatment by means of information that is more objective and covers the anamnesis more surely.

The storage of the healthcare data table 1221 enables outputting information related to the health condition and the degree of urgency of a medical treatment for the first user(patient), with anamnesis more surely covered, by means of healthcare data.

In an example of Fig. 3, the healthcare data table 1221 is stored in the storage unit 1022, but may be stored in a database (not shown) that a server provided separate from the healthcare device 1002 has. This assures that a server is configured separately from the healthcare device 1002, thereby the healthcare device 1002 being configured without the server and structured small and portable. A portable healthcare device 1002 can receive information that is more objective and covers the anamnesis more surely, even at a place far from a medical institution or the like in which a large healthcare device 1002 can be arranged, for example, an emergency medical care site, a nursing facility, etc. Further, a server can be configured without taking any account of portability of the healthcare device 1002, thereby a database storing more data being constructed. Then, the healthcare device 1002 can receive information that is more objective and covers the anamnesis more surely.

A server that is provided separate from the healthcare device 1002 may further have an authentication module (not shown) that can authenticate that a second user for whom a predetermined registration has been made is a user. This assures that the authentication module is configured integrally with the server, thereby authenticating only a second user (for example, a medical worker) who is present at a medical institution in which the server is arranged. This means that the authentication of the second user is more reliable. A reliable authentication is performed, thereby the first user(patient) providing the condition of the patient and/or the healthcare data correctly without less worries about the leakage of personal information. Therefore, the healthcare device 1002 can receive information that is more objective and covers the anamnesis more surely.

### [Health Condition Table 1222]

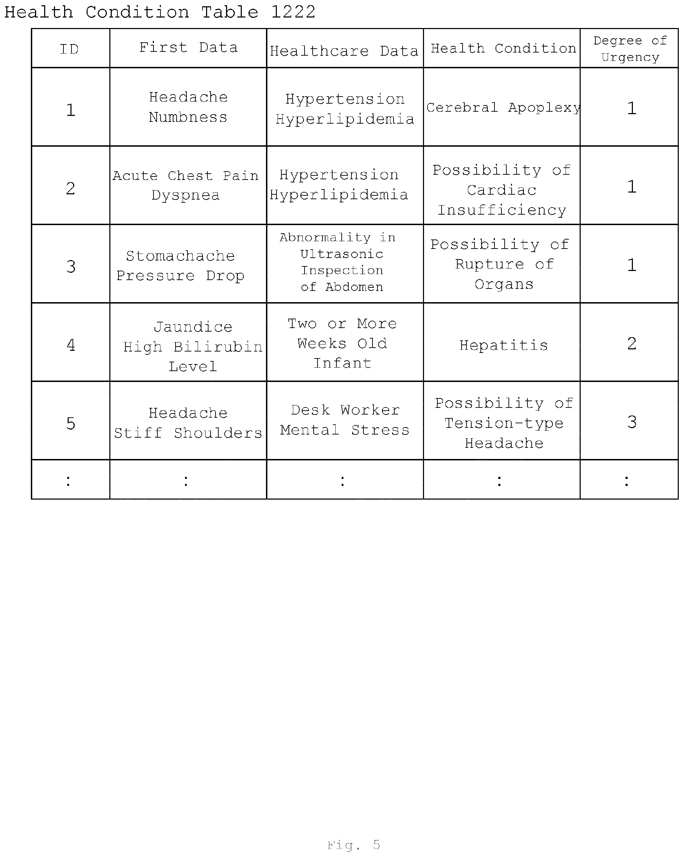

Fig. 5 is a view illustrating an example of a health condition table 1222. The health condition table 1222 shown in Fig. 5 stores information associated with an "ID" distinguishing a health condition, "First Data" related to the condition of the first user(patient), "Healthcare Data" of the patient, and "Health Condition" and "Degree of Urgency" related to the first data and/or the healthcare data. In the example shown in Fig. 5, the degrees of urgency "1", "2", and "3" are stored in order of high degree of urgency, i.e., in order of health conditions in which more prompt medical treatment should be performed.

There are no restrictions on a format of the first data related to the condition as long as it indicates a condition of the patient. There are no restrictions on a format of the healthcare data as long as it is data related to healthcare data of the patient. There are no restrictions on a format of the health condition as long as it indicates a health condition of the patient. There are no restrictions on a format of the degree of urgency as long as it indicates a degree of urgency related to a health condition.

The inclusion of an ID facilitates acquiring and storing healthcare data. The inclusion of first data related to a condition of the patient and healthcare data of the patient allows for the grasp of a health condition of the patient, and for a more accurate decision or the like of an order of a medical treatment policy, an order of an emergency transport, and a designated facility, by means of the condition of the patient and the healthcare data. The inclusion of the health condition and the degree of urgency enables the grasp of a health condition of the patient by the first data and the healthcare data, and for a more accurate decision or the like of an order of a medical treatment policy, an order of an emergency transport, and a designated facility.

For example, in the example shown in Fig. 5, data associated with an ID "1", first data "Headache" and "Numbness", healthcare data "Hypertension" and "Hyperlipidemia", a health condition "Cerebral Apoplexy", and a degree of urgency "1" is stored. This enables deciding the health condition and the degree of urgency related to the patient, in a triage processing to be referred to later, by using the facts that the first user(patient) with the conditions of "Headache" and/or "Numbness" has a possibility of cerebral apoplexy, and that the degree of urgency of cerebral apoplexy is 1, which is the highest degree of urgency.

The healthcare data preferably includes a possibility of injury, a possibility of disability, and/or a possibility of illness. For example, in the example shown in Fig. 5, an ID "2" is associated with a health condition "Possibility of Cardiac Insufficiency", which is a possibility of disability, an ID "3" with a health condition "Possibility of Rupture of Organs", which is a possibility of injury, and an ID "5" with a health condition "Tension-type Headache", which is a possibility of illness. This enables the healthcare device 1002 to output not only a correctly diagnosable health condition but also a possible health condition.

The storage of the health condition table 1222 enables the healthcare device 1002 to, from the health condition table 1222, acquire and output a health condition and a degree of urgency related to the patient associated with the condition and/or the healthcare data of the first user(patient) received by the healthcare device 1002.

The health condition table 1222 preferably can be stored by association of a health condition with healthcare information related to a healthcare measure to be performed to the first user and/or to healthcare advice for the patient, which is not an indispensable component. This enables the healthcare device 1002 to provide the patient a necessary healthcare measure and/or healthcare advice. Therefore, it is possible to provide the healthcare device 1002 that can decide more correctly the health condition and the degree of urgency of the patient related to the decision of a designated facility. Healthcare information related to the healthcare measure includes, for example, information related to a message for a medical worker who is in charge of a medical checkup at working space and/or school, information related to a selection of a medical examination department of a specialized category related to a medical treatment for the patient (for example, surgery, cardiovascular medicine, cardiovascular medicine, etc.), information related to a medical treatment policy for the patient, etc. Healthcare information related to the healthcare advice includes, for example, information related to advice on meals that the patient has at a restaurant or the like, information related to advice on a travel of the patient, etc.

### [Input Command Table 1223]

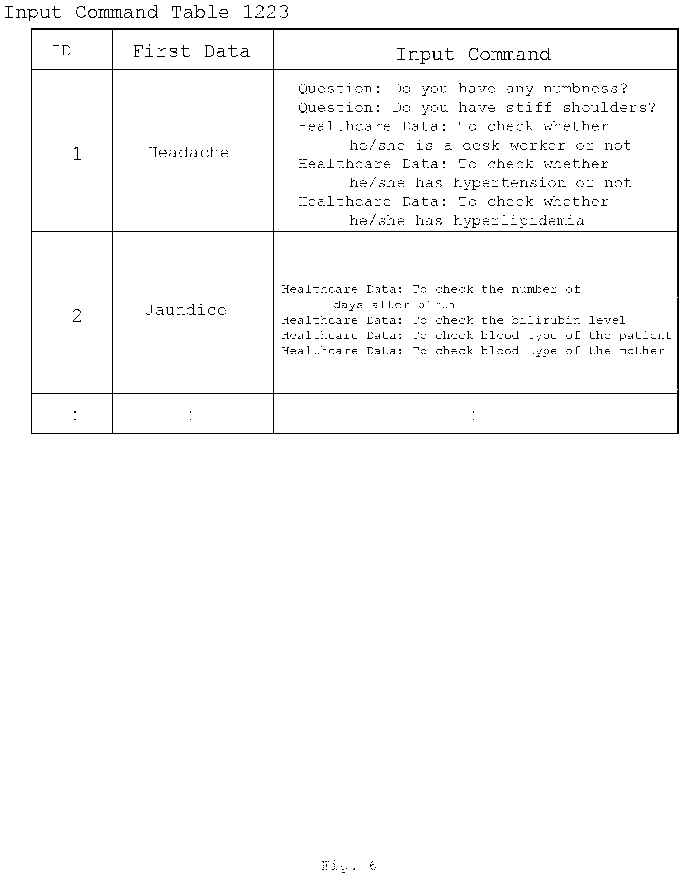

The storage unit 1022 preferably stores an input command table 1223. Fig. 6 is a view illustrating an example of the input command table 1223. The input command table 1223 shown in Fig. 6 stores information associated with an "ID" distinguishing an input command, "First Data" related to a condition of the first user(patient), and an "Input Command" commanding the first user(patient) to input an additional condition and/or additional healthcare data associated with the first user.

The inclusion of an ID facilitates acquiring and storing healthcare data. The inclusion of an input command enables the healthcare device 1002 to command the first user(patient) to input an answer to a necessary question and/or necessary healthcare data on the basis of first data. This assures that the healthcare device 1002 receives information that is more objective and covers the anamnesis more surely. Therefore, it is possible to provide the healthcare device 1002 that can decide more correctly the health condition and the degree of urgency of the patient related to the decision or the like of an order of a medical treatment for the patient, an order of an emergency transport, and a designated facility.

For example, in an example shown in Fig. 6, data associated with an ID "1", first data "Headache", and input commands "Question: Do you have any numbness?" "Question: Do you have stiff shoulders?" "Healthcare Data: To check whether he/she is a desk worker or not" "Healthcare Data: To check whether he/she has hypertension or not" "Healthcare Data: To check whether he/she has hyperlipidemia" is stored. This allows for a command for the first user(patient) having the condition of "headache" to input an answer to a necessary question and necessary healthcare data in a triage processing to be referred to later.

The storage of the input command table 1223 enables the input command unit 1214 to command the first user(patient) to input an answer to a necessary question and necessary healthcare data on the basis of the first data. This enables the healthcare device 1002 to receive information that is more objective and covers the anamnesis more surely. Therefore, it is possible to provide the healthcare device 1002 that can decide more correctly the health condition and the degree of urgency of the patient related to the decision or the like of an order of a medical treatment for the patient, an order of an emergency transport, and a designated facility.

### [Candidate Table 1224]

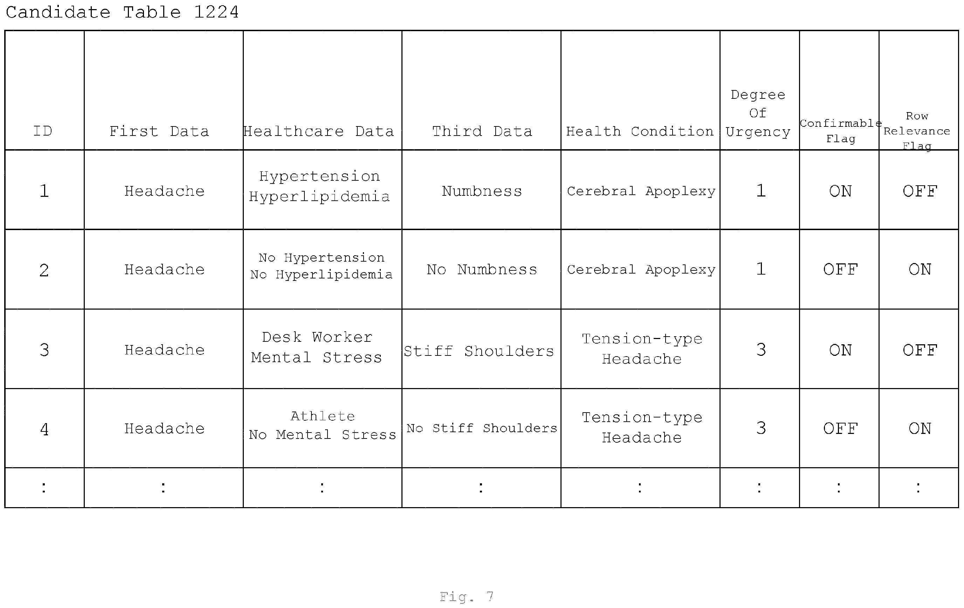

The storage unit 1022 preferably stores further a candidate table 1224 in addition to the input command table 1223. Fig. 7 is a view illustrating an example of the candidate table 1224. The candidate table 1224 shown in Fig. 7 stores information associated with an "ID" distinguishing a candidate of a health condition, "First Data" related to the condition of the first user(patient), "Healthcare Data" related to healthcare data of the patient, "Third Data" transmitted per input command, "Health Condition" and "Degree of Urgency" related to the first data, the healthcare data, and/or the third data, a "Confirmable Flag" indicating whether to be confirmable or not as a health condition of the patient, and a "Low Relevance Flag" indicating that the relevance with a condition of the patient is relatively low.

The inclusion of an ID facilitates acquiring and storing a candidate of healthcare data. The inclusion of the first data, the healthcare data, the third data, the health condition, the degree of urgency, and the confirmable flag enables the healthcare device 1002 to determine the presence/absence of a confirmable health condition to be defined as a disease or the like of the patient. Thus, the disease of the patient can be grasped quickly. The inclusion of the low relevance flag allows for a removal of a disease or the like that is predicted to have a relatively low relevance with a disease or the like of the patient. Therefore, it is possible to prevent the generation of a leakage of a severe disease presumed even if it unlikely occurs, and to grasp the degree of urgency accurately.

The storage of the candidate table 1224 enables a removal unit 1216 to remove a disease or the like that is predicted to have a relatively low relevance with a disease or the like of the patient, by means of a candidate of a health condition that a low relevance flag stored in the candidate table 1224 is ON. Further, the determination unit 1217 can determine the presence/absence of a confirmable health condition to be defined as a disease or the like of the first user, by means of a candidate of a health condition that a confirmable flag stored in the candidate table 1224 is ON.

### [Display Unit 1023]

The type of the display unit 1023 is not particularly limited. The display unit 1023 includes, for example, a monitor, a touch panel, a projector, a speaker capable of reproducing voice related to a health condition and a degree of urgency, and a video card occurring an external device to show a health condition and a degree of urgency, etc.

### [Input Unit 1024]

The type of the input unit 1024 is not particularly limited. The input unit 1024 includes, for example, a keyboard, a mouse, a touch panel, a software keyboard, a microphone recognizing voice, and a communication device receiving an input from an external device, etc.

### [Communication Unit 1025]

A communication unit 1025 has a device for allowing the healthcare device 1002 to communicate with first user terminal 1003 or other devices. The type of the device is not particularly limited and the device has, for example, a network card corresponding to Ethernet standards, and a device adaptable to Wi-Fi (Wireless Fidelity) conforming to IEEE 802.11, etc. Communication means are not particularly limited and communications are performed, for example, by means of wireless communication, wire communication, etc. Wireless communication protocols or standards are not particularly limited and may include at least one of, for example, Wireless Fidelity (Wi-Fi)^{®} such as IEEE 802.11 a/b/g/n/ax/ba/EHT or the like, Bluetooth^{®}, near field communication (NFC), radio frequency identifier (RFID), and mobile communication (for example, Long term Evolution (LTE), LTE-Advanced (LTE-A), and 5th Generation (5G). The module for wire communication includes, for example, a Universal Serial Bus (USB), and High-Multimedia Definition Interface (HDMI), and Recommended Standard 232 (RS-232).

### [First User Terminal 1003]

The type of the first user terminal 1003 used by the first user(patient), persons concerned of the patient including the family or the like of the patient, a worker for a specific company or at a medical institution such as a hospital, and/or another personnel who is allowed for an access to the system 1001 is not particularly limited and the first user terminal 1003 includes, for example, various terminals such as a personal computer, a tablet, a smartphone, etc. The first user terminal 1003 is configured to communicate with the healthcare device 1002 via a network 1004. Further, the first user terminal 1003 is configured so as to transmit first data and third data to the healthcare device 1002, to store a program receiving an input command and second data, and to perform such program.

The first user terminal 1003 may include a sensor (not shown) configured so as to acquire image data of the first user(patient) to transmit it to the healthcare device 1002, which is not an indispensable component. The sensor may be an image acquisition camera acquiring at least one image of at least one part of the body of the patient. The at least one acquired image may be a still image or a frame of a video. When the sensor acquires at least one image, the sensor may transmit image data acquired by the healthcare device 1002. The sensor may further analyze a characteristic of at least one image data that is acquired.

In the case that the sensor acquires a plurality of images, the sensor may analyze the vibration degree of the first user (i.e. a change of positions of a detected part included in a plurality of images). The vibration degree may be associated with an extent of move of a predetermined period, a person, an individual, or of a body part of a subject (for example, spasms of an eye, a raise of an eyebrow, a dilation of pupils of eyes, a tension of facial muscles, etc.). The vibration degree may be decided on the basis of a comparison with a plurality of images acquired at different timing. After the vibration degree being established or decided, the sensor may decide a condition of the first user. A great vibration degree associated with a single body part or a plurality of body parts may show an emotional condition, a physical condition, or a mental condition. The use of various conditions related to a facial expression of the first user may derive an index of aggression, tension, normality, rest/rest, or fatigue.

A sensor can preferably acquire image data at different timing. The decision unit 1212 equivalent to a control unit deciding an output related to a health condition and a degree of urgency can perform the grasp of a pace of a symptom, and the diagnosis of a disease that a symptom changes depending on time zones of a day, or the like, by comparing each of a plurality of image data of the first user(patient) acquired at different timing, on the basis of the first data including a plurality of image data and of the healthcare data, when deciding second data including information related to the health condition and the degree of urgency, by the fact that the sensor can acquire image data at different timing. Therefore, it is possible to provide the system 1001 that can decide more correctly the health condition and the degree of urgency of the patient related to a decision or the like of an order of a medical treatment for the patient, an order of an emergency transport, and a designated facility.

At least one image data may be acquired by a still camera or a video camera. The still camera or the video camera may be a surveillance camera arranged in a firstly decided area (for example, a street, or a platform of a station), or a detection camera arranged in a secondly decided area (for example, an immigration control area in an airport). Further, at least one image data may be acquired by a device with a photographing and/or video-recording function.

The sensor may be a sensor, such as an infrared sensor, that can acquire a thermal image of the first user(patient). By the fact that the sensor is a sensor that can acquire a thermal image, the decision unit 1212 equivalent to a control unit deciding an output related to a health condition and a degree of urgency can measure a surface temperature of the skin of the patient by means of first data including thermal image data, and can decide second data on the basis of various objective information of the patient, such as peripheral circulatory insufficiency of the patient, the presence/absence of right and left difference of a body temperature caused by a paralysis, a dermatosis, and/or a temperature difference from a peripheral part like hemangioma or the like, etc.

### [Network 1004]

The type of the network 1004 is not particularly limited and the network 1004 includes, for example, a personal area network, a local area network, an intranet, an extranet, the Internet, a Wi-Fi network, and a bus network conforming to Universal Serial Bus (USB), and a network obtained by combining a plurality of networks including these networks.

### [Main flowchart of image distribution]

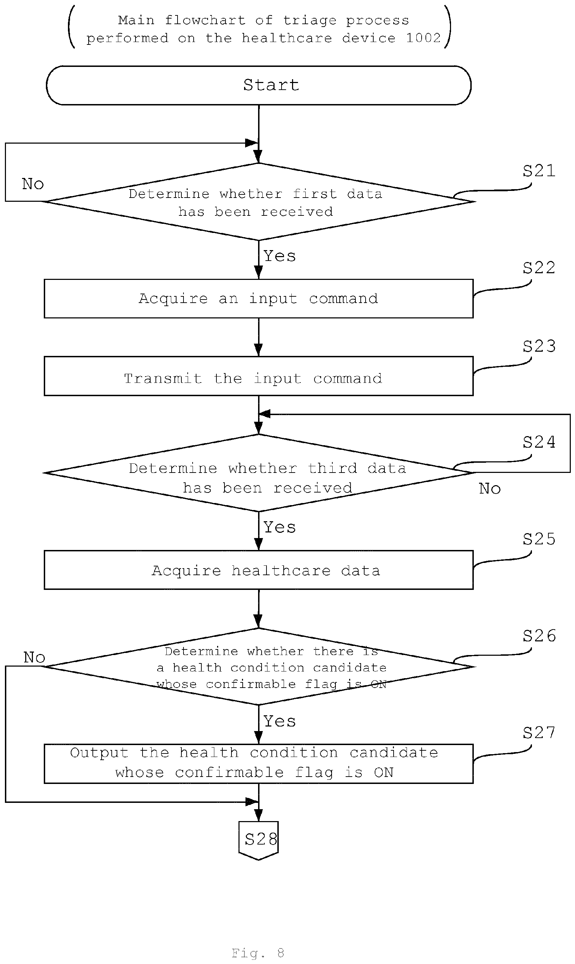

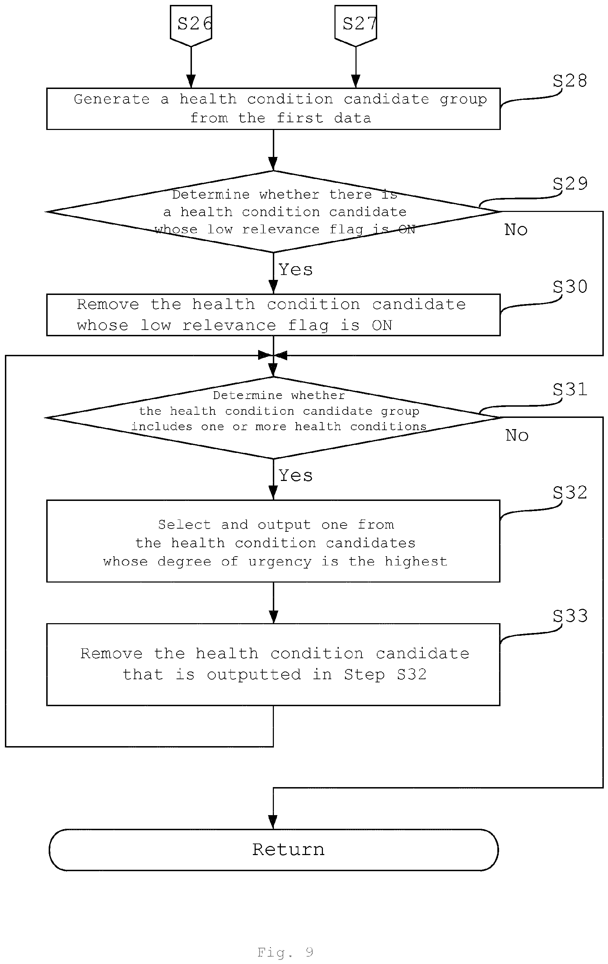

Figs. 8 and 9 are an example of a main flowchart illustrating the procedure of the processing to decide a health condition and a degree of urgency thereof related to the first user in the present embodiment (hereinafter referred to also as a triage process). In the following, with reference to Figs. 8 and 9, description is given of an example of a preferred procedure of the triage process performed by the healthcare device 1002.

### [Step S21: Determine whether first data has been received]

The processing unit 1021 executes the reception unit 1211 in cooperation with the storage unit 1022 and the communication unit 1025 to determine whether first data has been received including a condition of the first user(patient) from the first user terminal 1003 (Step 21).

In Step 21, by determining whether the first data has been received, the triage process can be performed after surely receiving the first data including a condition of the patient.

The first data preferably includes image data of the first user(patient) acquired by a sensor (not shown) provided separate from the healthcare device 1002. The sensor may be a sensor that the first user terminal 1003 has. By the fact that the first data includes the image data of the patient, it is possible to receive image data of the patient that is objective data excluding subjectivity of an operator. Therefore, it is possible to provide the system 1001 that can decide more correctly the health condition and the degree of urgency of the patient related to the decision or the like of an order of a medical treatment for the patient, an order of an emergency transport, and a designated facility.

The image data of the first user(patient) preferably includes image data acquired at different timing. This assures that the decision unit 1212 equivalent to a control unit deciding an output related to a health condition and a degree of urgency can perform the grasp of a pace of a symptom, and the diagnosis of a disease that a symptom changes depending on time zones of a day, or the like, by comparing each of a plurality of image data of the first user(patient) acquired at different timing, on the basis of the first data including a plurality of image data and of the healthcare data, when deciding second data including information related to the health condition and the degree of urgency, by the fact that the sensor can acquire image data at different timing.

The image data of the first user(patient) preferably includes thermal image data of the first user(patient) acquired by an infrared sensor or the like. The inclusion of thermal image data assures that the decision unit 1212 equivalent to a control unit deciding an output related to a health condition and a degree of urgency can measure a surface temperature of the skin of the patient by means of first data including thermal image data, and can decide second data on the basis of various objective information of the patient, such as peripheral circulatory insufficiency of the patient, the presence/absence of right and left difference of a body temperature caused by a paralysis, a dermatosis, and/or a temperature difference from a peripheral part like hemangioma or the like, etc.

If the first data has been received, the processing unit 1021 shifts the processing to Step S22. If the first data has not been received, the processing unit 1021 shifts the processing to Step S21.

The healthcare device 1002 preferably performs the processing related to an input command performed in Steps S21 to S24, but does not need to perform the processing related to an input command. For example, the processing unit 1021 may shift the processing to Step S25 in the case that the first data has been received.

### [S22: Acquire an input command]

The processing unit 1021 executes the input command unit 1214 in cooperation with the storage unit 1022 to acquire an input command associated with one or more of conditions of the first user(patient) included in the first data received from the input command table 1223 in Step S21 (Step S22). The processing unit 1021 shifts the processing to Step S23.

In Step S22, by the acquisition of the input command, it is possible to transmit an input command for the first user terminal 1003 to input an answer to a question and/or necessary healthcare data. This prevents a leakage of a question content caused by the subjectivity of a second user, and enables performing an objective question to a patient.

### [Step S23: Transmit the input command]

The processing unit 1021 executes the input command unit 1214 in cooperation with the storage unit 1022 and the communication unit 1025 to transmit the input command acquired in Step S22 to the first user terminal 1003 (Step S23). The processing unit 1021 shifts the processing to Step S24.

In Step S23, the transmission of the input command to the first user terminal 1003 allows for commanding an input of an answer to a necessary question and/or of necessary healthcare data to the first user(patient) operating the first user terminal 1003, persons concerned of the patient including the family or the like of the patient, a worker for a specific company or at a medical institution such as a hospital, and/or another personnel who is allowed for an access to the system 1001.

### [Step S24: Determine whether third data has been received]

The processing unit 1021 executes the reception unit 1211 in cooperation with the storage unit 1022 and the communication unit 1025 to determine whether third data has been received that includes an answer to a necessary question and/or necessary healthcare data related to the first user(patient) from the first user terminal 1003 (Step S24). If the third data has been received, the processing unit 1021 shifts the processing to Step S25. If the third data has not been received, the processing unit 1021 shifts the processing to Step S24.at

In Step 24, by determining whether the third data has been received, the triage process can be performed after surely receiving the third data including an answer to a necessary question and/or necessary healthcare data.

### [Step S25: Acquire healthcare data]

The processing unit 1021 executes the decision unit 1212 in cooperation with the storage unit 1022 to acquire healthcare data related to the first user(patient) from the healthcare data table 1221 (Step S25).

In Step S25, the acquisition of the healthcare data enables the output unit 1213 to output information related to a health condition and a degree of urgency of a medical treatment for the patient, with anamnesis more surely covered, by means of healthcare data.

The processing unit 1021 shifts the processing to Step S26. The healthcare device 1002 preferably performs the processing that is performed by the execution of the determination unit 1217 in Steps S26 and S27 to be referred to later and that is related to a confirmable health condition, but does not need to perform the processing related to a confirmable health condition. For example, the processing unit 1021 may shift the processing to Step S28.

### [Step S26: Determine whether there is a health condition candidate whose confirmable flag is ON]

The processing unit 1021 executes the determination unit 1217 in cooperation with the storage unit 1022 to determine whether the candidate table 1224 stores all conditions included in the first data received in Step S21, all conditions and/or healthcare data included in the third data received in Step S24, the healthcare data acquired in Step S25, and a health condition associated with a confirmable flag "ON" and a degree of urgency related to such health condition (hereinafter referred to also as "a health condition candidate whose confirmable flag is ON")(Step S26). If the candidate table 1224 stores the health condition candidate whose confirmable flag is ON, the processing unit 1021 shifts the processing to Step S27. If the candidate table 1224 doesn't store the health condition candidate whose confirmable flag is ON, the processing unit 1021 shifts the processing to Step S28.

The processing performed in Step S26 enables the determination unit 1217 to determine the presence/absence of a confirmable health condition that is confirmable as a disease or the like of the first user by means of the first data, the third data, and the healthcare data. Then, if a health condition candidate whose confirmable flag is ON, i.e., a disease that can be confirmed as a disease of the patient, has been found, it is possible to output a confirmable disease and degree of urgency thereof. Further, if a disease that can be confirmed as a disease of the patient has not been found, the second data is decided by means of a health condition candidate group to be referred to later that is removed by the removal unit 1216 and of the healthcare data. Therefore, it is possible to make the most use of present information, to minimize the risk of a leakage in a presumed severe disease even if it unlikely occurs, and to predict the degree of urgency as accurately as possible.

### [Step S27: Output the health condition candidate whose confirmable flag is ON]

The processing unit 1021 executes the decision unit 1212 in cooperation with the storage unit 1022 and the communication unit 1025 to acquire one or several health condition candidates whose confirmable flag is ON from the candidate table 1224, then to transmit a command to display the acquired one or several health condition candidates whose confirmable flag is ON to the first user terminal 1003 (Step S27). The processing unit 1021 shifts the processing to Step S28.

By acquiring one or several health condition candidates whose confirmable flag is ON from the candidate table 1224 then transmitting a command to display the acquired one or several health condition candidates whose confirmable flag is ON to the first user terminal 1003, when a disease that can be confirmed as a disease of the patient, the decision unit 1212 equivalent to a control unit deciding a health condition or the like can decide the second data, by means of this confirmable disease. Thus, it is possible to grasp quickly the disease of the patient.

### [Step S28: Generate a health condition candidate group from the first data]

The processing unit 1021 executes the generation unit 1215 in cooperation with the storage unit 1022 to acquire one or several health conditions and degrees of urgency related to such health conditions, that are associated with any condition included in the first data received in Step S21, from the health condition table 1222, then to generate a health condition candidate group including the health condition candidate associating the acquired one or several health conditions with the degrees of urgency (Step S28).

In Step S28, by acquiring one or several health conditions including any condition included in the first data from the health condition table 1222 then generating a health condition candidate group including a health condition candidate associating the acquired one or several health conditions with the degrees of urgency, the generation unit 1215 can generate a health condition candidate group including presumed diseases without omission, on the basis of the condition of the first user(patient) and of the healthcare data.

In Step S28, the processing unit 1021 may execute a mental condition determination unit (not shown) determining a mental condition of the first user on the basis of a plurality of image data, in cooperation with the storage unit 1022, to determine a mental condition of the first user(patient) on the basis of a plurality of image data acquired at different timing and included in the first data that is received at least Step S21.

This assures that, on deciding the second data that includes information related to the health condition and the degree of urgency, the decision unit 1212 equivalent to a control unit of the healthcare device 1002 can determine a mental condition by means of image data of the first user(patient) acquired at different timing, and can decide not only a physical disease but also a mental disease of the patient. Further, on deciding the second data, such control unit considers the synergistic effect of the physical disease and the mental condition to decide the degree of urgency of the health condition. Therefore, it is possible to provide the system 1001 that can decide more correctly the health condition and the degree of urgency of the patient related to the decision or the like of an order of a medical treatment for the patient, an order of an emergency transport, and a designated facility.

The processing unit 1021 shifts the processing to Step S29. The healthcare device 1002 preferably performs the processing that is performed by the execution of the removal unit 1216 in Steps S29 and S30 to be referred to later and that is related to a disease or the like predicted to have a relatively low relevance to a disease or the like of the first user, but does not need to perform the processing related to a disease or the like that is predicted to have a relatively low relevance to a disease or the like of the first user. For example, the processing unit 1021 may shift the processing to Step S31.

### [Step S29: Determine whether there is a health condition candidate whose low relevance flag is ON]

The processing unit 1021 executes the removal unit 1216 in cooperation with the storage unit 1022 to determine whether the candidate table 1224 stores all conditions included in the first data received in Step S21, all conditions and/or healthcare data included in the third data received in Step S24, the healthcare data acquired in Step S25, and a health condition associated with a low relevance flag "ON" (hereinafter referred to also as "a health condition candidate whose low relevance flag is ON")(Step S29). If the candidate table 1224 stores the health condition candidate whose low relevance flag is ON, the processing unit 1021 shifts the processing to Step S30. If the candidate table 1224 doesn't store the health condition candidate whose low relevance flag is ON, the processing unit 1021 shifts the processing to Step S31.

In the case of the presence of a disease or the like that is predicted to have a relatively low relevance to a disease or the like of the first user, the processing performed in Step S29 enables performing the processing to remove it.

### [Step S30: Remove the health condition candidate whose low relevance flag is ON]

The processing unit 1021 executes the removal unit 1216 in cooperation with the storage unit 1022 to remove the health condition candidate that is stored in the candidate table 1224 and has the same health condition as the health condition candidate whose low relevance flag is ON, from the health condition candidate group generated in Step S28 (Step S30). The processing unit 1021 shifts the processing to Step S31.

From the health condition candidate group generated in Step S28, the removal unit 1216 removes the health condition candidate that has the same health condition as the health condition candidate whose low relevance flag is ON, thereby removing a disease or the like that is predicted to have a relatively low relevance to a disease or the like of the first user, by means of the third data acquired from the input of an answer to a necessary question commanded on the basis of the condition of the patient and from the condition of the patient, and of the healthcare data covering more surely the anamnesis from which the patient has suffered.

On grasping the degree of urgency, it is important to presume a severe disease even if it unlikely occurs, and a leakage of a possible disease is inadmissible.

The generation unit 1215 can generate a health condition candidate group including a possible disease without omission on the basis of the health condition of the first user(patient) and the healthcare data, by performing the processing performed by the execution of the determination unit 1217 in Steps S26 and S27 and related to a confirmable health condition and the processing performed by the execution of the removal unit 1216 in Steps S29 and S30 to be referred to later and related to a disease or the like that is predicted to have a relatively low relevance to a disease or the like of the first user, in addition to the processing of generating the health condition candidate group performed by the execution of the generation unit 1215 in Step S28. Then, the removal unit 1216 removes a disease or the like predicted to have a relatively low relevance to a disease or the like of the first user, by means of the third data acquired from the input of an answer to a necessary question commanded on the basis of the condition of the patient and from the condition of the patient, and of the healthcare data covering more surely the anamnesis from which the patient has suffered. Further, the determination unit 1217 determines the presence/absence of a confirmable health condition that can be confirmed as a disease or the like of the first user, by means of the aforementioned third data and the healthcare data.

Then, if the determination unit 1217 finds a disease that can be confirmed as a disease of the patient, the decision unit 1212 equivalent to a control unit deciding a health condition or the like can decide the second data by means of this confirmable disease. Thus, it is possible to grasp the disease of the patient. Additionally, the decision unit 1212 can decide the second data, not only by a confirmable disease but also by a health condition candidate group removed by the removal unit 1216 and by healthcare data. Therefore, it is possible to avoid occurring a leakage in a presumed severe disease even if it's unlikely then to grasp a degree of urgency accurately.

On the other hand, if the determination unit 1217 doesn't find a disease that can be confirmed as a disease of the patient, the decision unit 1212 can decide the second data by means of the health condition candidate group removed by the removal unit 1216 and of the healthcare data. Therefore, it is possible to make the most use of present information, to minimize the risk of a leakage in a presumed severe disease even if it unlikely occurs, and to predict the degree of urgency as accurately as possible.

### [Step S31: Determine whether the health condition candidate group includes one or more health conditions]

The processing unit 1021 executes the output unit 1213 in cooperation with the storage unit 1022 to determine whether the health condition candidate group includes one or more health conditions (Step S31). If one or more health conditions are included, the processing unit 1021 shifts the processing to Step S32. If one or more health conditions are not included, the processing unit 1021 shifts the processing to Step S21 and repeats from Step 21 to Step S33.

By determining whether the health condition candidate group includes one or more health conditions, it is possible to perform the processing of outputting the health condition candidate included in the health condition candidate group, only if the health condition candidate group includes one or more health conditions.

### [Step S32: Select and output one from the health condition candidates whose degree of urgency is the highest]

The processing unit 1021 executes the output unit 1213 in cooperation with the storage unit 1022 and the communication unit 1025 to transmit a command to select and output one from the health condition candidates whose degree of urgency is the highest, of the health condition candidates included in the health condition candidate group, to the first user terminal 1003 (Step S32). The processing unit 1021 shifts the processing to Step S33.

It is possible to output information related to the health condition of the patient and the degree of urgency of a medical treatment, by transmitting a command to select and output one from the health condition candidates whose degree of urgency is the highest, of the health condition candidates included in the health condition candidate group, to the first user terminal 1003.

### [Step S33: Remove the health condition candidate that is outputted in Step S32]

The processing unit 1021 executes the output unit 1213 in cooperation with the storage unit 1022 and the communication unit 1025 to remove the health condition candidate that is outputted in Step S32, from the health condition candidate group (Step S33). The processing unit 1021 shifts the processing to Step S31.

It is possible to avoid outputting the same health condition candidate for a plurality of times, by removing the health condition candidate that is outputted in Step S32, from the health condition candidate group.

The aforementioned procedure from Step S21 to Step S33 enables the healthcare device 1002 to receive information excluding subjectivity of an operator as much as possible, thereby, by adding such information and information covering more surely the anamnesis from which the patient has suffered, providing the healthcare device 1002 that can perform more correctly the grasp of a health condition and a degree of urgency of a patient, and the decision or the like of an order of a medical treatment for the patient, an order of an emergency transport, and a designated facility.

In the flow of the processing exemplified by means of Figs. 8 and 9, while receiving information related to the first user from the first data terminal 1003 in Step S21 and Step 24, the healthcare device 1002 of the present embodiment may receive information that is related to the first user and that a second user inputs by means of the input unit 1024. Further, while transmitting a command to display various information on the first data terminal 1003 operated by a second user in Step S23, Step 7 and Step 32, the healthcare device 1002 of the present embodiment may display such information on the display unit 1023. This enables the healthcare device 1002 to execute the triage processing even when connection to the network 1004 is disabled.

### [Usage example of system]

In the following, description is given of a usage example of the system 1001 according to the present embodiment.

According to the present embodiment, the healthcare device 1002 is configured so as to connect with the first user terminal 1003 via the network 1004.

### [Transmission of a condition of a patient]

Firstly, a second user using a healthcare device 1002 transmits first data including a condition of a first user that is a patient, via a first user terminal 1003. The healthcare device 1002 executes a reception unit 1211 to receive the first data.

### [Input an answer or the like to a question]

The healthcare device 1002 executes an input command unit 1214 to acquire an input command to command an input of answers to one or several necessary questions associated with the first data stored in an input command table 1223, and/or of necessary healthcare data. Then, the healthcare device 1002 transmits one or several input commands acquired by the first user terminal 1003 that has transmitted the first data. The first user terminal 1003 receives these input commands to display for the second user. The second user inputs third data including answers to the one or several necessary questions and/or the necessary healthcare data, via the first user terminal 1003, to transmit the input third data to the healthcare device 1002. The healthcare device 1002 receives the third data.

### [Output a confirmable health condition candidate]

The healthcare device 1002 executes a determination unit 1217 to command, if a candidate table 1224 stores one or several health conditions that can be confirmed by means of the first data, the healthcare data, and of the third data, a display of the one or several confirmable health conditions and a degree of urgency related to such health conditions to shift to the processing of displaying the health condition and the degree of urgency. If the candidate table 1224 does not store the one or several confirmable health conditions, the healthcare device 1002 shifts to the processing of displaying the health condition and the degree of urgency.

### [Display the health condition and the degree of urgency]

The healthcare device 1002 executes a generation unit 1215 to acquire a health condition associated with first data stored in a health condition table 1224, and a degree of urgency thereof to generate a health condition candidate group including a disease presumed by means of the first data without omission. The healthcare device 1002 executes a removal unit 1216 to remove, if a candidate table 1224 stores one or several health condition candidates predicted to have a relatively low relevance with a disease or the like of the first user and associated with the first data, the healthcare data, and with the third data, such health condition candidates from the health condition candidate group. The healthcare device 1002 executes an output unit 1213 to command the first user terminal 1003 to display one or several health conditions included in the health condition candidate group, and degrees of urgency related to such health conditions, in the order of the highest degree of urgency.

Therefore, the healthcare device 1002 can receive information excluding subjectivity of an operator as much as possible. Then, by adding such information and information covering more surely anamnesis from which a patient has suffered, it is possible to provide the healthcare device 1002 that can grasp more correctly a health condition and a degree of urgency of a patient exemplified by a predicted disease, thereby a medical examination department of a specialized category related to a selected medical examination or the like, and can select more correctly a necessary measure exemplified by a decision or the like of an inspection necessary for the patient, a medical treatment policy, a degree of urgency of a consultation, an order of a medical treatment for the patient, an order of an emergency transport, and a designated facility, on the basis of the health condition and the degree of urgency of the patient.

### [Modified example] A healthcare device 1002 performing machine learning]

In the system 1001 of the second embodiment, the processing of generating the health condition candidate group is performed by the acquisition of a health condition associated with first data or the like and of a degree of urgency thereof, from the health condition table 1222, but not limited thereto. The processing of generating a health condition candidate group of the present invention may be performed on the basis of a neural network that has performed machine learning related to a health condition and to a degree of urgency of such health condition by means of first data and third data.

A healthcare device 1002 of the present modified example is further provided with a machine learning unit (not shown) that causes a neural network to perform machine learning related to a health condition and a degree of urgency of such health condition by means of first data and third data. Further, a storage unit 1022 of the present modified embodiment is configured so as to store the aforementioned neural network (not shown).

The healthcare device 1002 of the present modified example generates a health condition candidate group in Step S28 shown in Fig. 9, on the basis of the neural network that performs the machine learning related to the health condition and the degree of urgency of such health condition by means of the first data and the third data. This allows for deciding a health condition of a patient that is conjectured from a condition of the patient, healthcare data, and/or from question, on the basis of the neural network that performs the machine learning.

The healthcare device 1002 of the present modified example causes the neural network to perform the machine learning related to the health condition and the degree of urgency of such health condition. Timing to cause the neural network to perform the machine learning is not limited and, for example, before performing Step S31 shown in Fig. 9, in cooperation with the processing unit 1021 and the storage unit 1022, the healthcare device 1002 may execute the machine learning unit that causes the neural network to perform the machine learning related to the health condition and the degree of urgency of such health condition by means of the first data and of the third data, thereby causing the neural network to perform the machine learning related to the health condition and the degree of urgency of such health condition.

By causing the neural network to perform the machine learning related to the health condition and the degree of urgency of such health condition, the healthcare device 1002 can perform the machine learning of the neural network based on the conditions of the patient that are stored, the healthcare data, and/or of questions. Further, based on the machine learning, the healthcare device 1002 can find a new relevance between the conditions of the patient, the healthcare data, and/or the questions, and a disease, thereby increasing the accuracy of deciding a disease and a degree of urgency. By using machine learning, without spending labor and time for developers or the like of the healthcare device 1002 to refer to enormous first data and third data in order to search a relevance between these data and a disease, the healthcare device 1002 can decide what disease the patient has and how urgent it is.

A person skilled in the art should understand that variations and combinations of the aforementioned features are not an alternative or a substitute and can be combined within the intended scope of the disclosure in order to form further embodiments.

As understood by the person skilled in the art, every embodiment may be combined with another embodiment or some embodiments for use. The present disclosure is applicable to a medical diagnosis (for example, a medical diagnosis performed in perinatal period) and/or an operation at hospital.

### [Description of the reference numerals]

- 1: System
- 2: Healthcare Device
- 21: Reception Unit
- 22: Control Unit
- 23: Output Module
- 24: Transmission Module
- 3: Server
- 31: Database
- 31A: Database
- 31B: Database
- 32: Communication Module
- 4: Authentication Device
- 41: Authentication Module
- 42: Database
- 5: First Device
- 6: Second Device
- 7: Third Device
- 8: Fourth Device
- 1001: System
- 1002: Healthcare Device
- 1021: Processing Unit
- 1211: Reception Unit
- 1212: Decision Unit
- 1213: Output Unit
- 1214: Input Command Unit
- 1215: Generation Unit
- 1216: Removal Unit
- 1217: Determination Unit
- 1022: Storage Unit
- 1221: Healthcare Data Table
- 1222: Health condition Table
- 1223: Input Command Table
- 1224: Candidate Table
- 1023: Display Unit
- 1024: Input Unit
- 1025: Communication Unit
- 1003: First User Terminal
- 1004: Network

## Claims

1. A healthcare device comprising:
a reception unit that receives first data related to at least one condition associated with a first user;
a control unit that decides second data on the basis of at least the first data and healthcare data; and
an output module that outputs the second data,
the second data comprising first information and second information,
the first information comprising at least one health condition of the first user,
the second information comprising a degree of urgency with respect to each of the at least one health condition.

2. The healthcare device according to claim 1, wherein the at least one health condition comprises a possibility of injury, a possibility of disability, and/or a possibility of illness.

3. The healthcare device according to claim 1 or 2, wherein the healthcare data is data that the reception unit receives from the outside of the healthcare device.

4. The healthcare device according to any of claims 1 to 3,
wherein the reception unit receives the first data when a second user for whom a predetermined registration has been made succeeds in an authentication of a user.

5. The healthcare device according to claim 4, wherein the authentication comprises face authentication, fingerprint authentication, iris authentication, voiceprint authentication, vein authentication, card authentication, or a combination thereof.

6. The healthcare device according to claim 4 or 5, wherein the first user is the same as the second user.

7. The healthcare device according to any of claims 1 to 6, wherein the healthcare data comprises: perinatal period healthcare data related to perinatal period healthcare associated with the first user; and/or infant healthcare data associated with an infant who is a child of the first user.

8. The healthcare device according to any of claims 1 to 7 further comprising an input command unit that commands an input of third data on the basis of the first data,
the third data comprising: an additional condition associated with the first user; and/or the healthcare data of addition,
the reception unit receiving the third data that is input according to the command,
the control unit deciding the second data on the basis of the third data in addition to the first data and the healthcare data.

9. The healthcare device according to claim 8 further comprising:
a generation unit that generates a health condition candidate group comprising one or more health condition candidates that are possible as health conditions of the first user, on the basis of the first data and the healthcare data;
a removal unit that removes zero or more of the health condition candidates with a relatively low relevance to the health conditions of the first user, from one or more of the health condition candidates that is included in the health condition candidate group, on the basis of the first data, the healthcare data, and the third data; and
a determination unit that determines the presence/absence of one or more confirmable health conditions that can be confirmed as the health conditions of the first user, on the basis of the first data, the healthcare data, and the third data,
the control unit:
deciding the second data on the basis of the health condition candidate group removed by the removal unit, the healthcare data, and of the one or more confirmable health conditions, if the determination unit determines the presence of the confirmable health conditions; and
deciding the second data on the basis of the health condition candidate group removed by the removal unit and of the healthcare data, if the determination unit determines the absence of the confirmable health conditions.

10. The healthcare device according to claim 8 or 9 further comprising a machine learning unit that causes a neural network to perform machine learning related to the health condition and the degree of urgency of the health condition by means of the first data and the third data,
the control unit deciding the second data on the basis of the neural network in addition to the first data, the healthcare data, and the third data.

11. The healthcare device according to any of claims 1 to 10,
wherein the second data further comprises information related to a healthcare measure to be performed to the first user, and/or to healthcare advice for the first user.

12. The healthcare device according to any of claims 1 to 11,
wherein the healthcare data comprises gene data of the first user, data related to mind of the first user, and/or data related to a living environment of the first user.

13. A system configured to comprise:
the healthcare device according to any of claims 1 to 12; and
an authentication module that is provided separate from the healthcare device and can authenticate that a second user for whom a predetermined registration has been made is a user,
the reception unit receiving the first data when the authentication module authenticates the user to be the second user.

14. A system configured to comprise:
the healthcare device according to any of claims 1 to 12; and a server that is provided separate from the healthcare device,
the server having a database that stores the healthcare data,
the reception unit receiving the healthcare data from the database.

15. The system according to claim 14, wherein the server further has an authentication module that can authenticate that a second user for whom a predetermined registration has been made is a user.

16. The system configured to comprise:
the healthcare device according to any of claims 1 to 12; and
a sensor that is provided separate from the healthcare device,
the sensor acquiring at least one image data of the first user and outputting the at least one acquired image data as a part or all of the first data on the reception unit.

17. The system according to claim 16, wherein there is a plurality of the image data,
the sensor acquiring the plurality of the image data at different timing.

18. The system according to claim 17, wherein the control unit of the healthcare device has a mental condition determination unit that determines a mental condition of the first user on the basis of the plurality of the image data,
the control unit deciding the second data on the basis of the first data and the mental condition.

19. The system according to any of claims 16 to 18, wherein one or more image data of the at least one image data comprises thermal image data.

20. A method for using a healthcare device comprising:
a step of a reception unit receiving first data related to at least one condition associated with a first user;
a step of a control unit deciding second data on the basis of at least the first data and healthcare data; and
a step of an output module outputting the second data,
the second data comprising first information and second information,
the first information comprising at least one health condition of the first user,
the second information comprising a degree of urgency with respect to each of the at least one health condition.

21. A program causing a healthcare device to perform:
a reception step of receiving first data related to at least one condition associated with a first user;
a control step of deciding second data on the basis of at least the first data and healthcare data; and
an output step of outputting the second data,
the second data comprising first information and second information,
the first information comprising at least one health condition of the first user,
the second information comprising a degree of urgency with respect to each of the at least one health condition.
